# EUROPEAN PATENT APPLICATION

(11) **EP 1 975 159 A1**
(43) Date of publication of application: **01.10.2008**
(21) Application number: 07090059.2
(22) Date of filing: 27.03.2007
(51) Int. Cl.: C07D 209/88, C07D 401/12, C07D 405/12, A61K 31/403, A61P 5/08, A61P 19/10

(54) **2,3,4,9-Tetrahydro-1H-carbazoles**

(71) Applicant: Bayer Schering Pharma Aktiengesellschaft, 13353 Berlin (DE)
(72) Inventor: Wortmann, Lars, 10435 berlin (DE); Koppitz, Marcus, 10115 Berlin (DE); Muhn, Peter, 13465 Berlin (DE); Frenzel, Thomas, 65719 Hofheim (DE); Liesener, Florian, Peter, 83308 Trostberg (DE); Schrey, Anna, Katharina, 10179 Berlin (DE); Kühne, Ronald, 12683 berlin (DE)

(57) **Abstract**

The present invention relates to 2,3,4,9-tetrahydro-1 H-carbazoles of the general formula I, 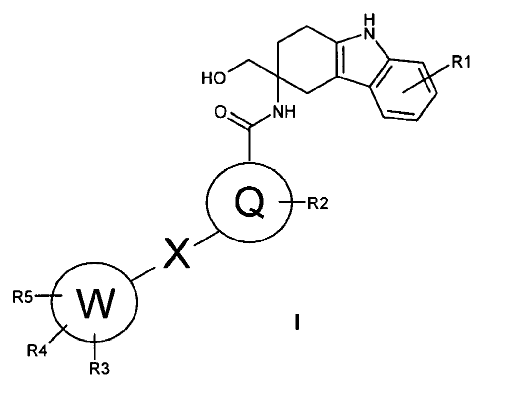 in which Q, X, W, R1, R2, R3, R4 and R5 have the meaning as defined in the description.

The compounds according to the invention are effective FSH antagonists and can be used for example for fertility control in men or in women, or for the prevention and/or treatment of osteoporosis.

## Description

The present invention relates to novel 2,3,4,9-tetrahydro-1 H-carbazoles with FSH-receptor antagonist activity. The present invention also relates to a process for their preparation, pharmaceutical compositions comprising the compounds according to the invention, and the use thereof for fertility control in men or women, for the treatment and/or prevention of osteoporosis.

Follicle-stimulating hormone (FSH) and luteinizing hormone (LH) are together responsible for the control of male and female fertility and of the production of sex steroids.

In the female mammal, FSH controls the early ripening of ovarian primary follicles and the biosynthesis of sex steroids. In the advanced stage of differentiation (preantral follicles), the influence of LH becomes increasingly important for further development of the follicles until ovulation occurs.

In male mammals, FSH is primarily responsible for the differentiation and stimulation of Sertoli cells. Their function consists of assisting spermatogenesis on many levels. LH is primarily responsible for stimulating the Leydig cells and thus androgen production. FSH, LH and TSH (thyrotropic hormone) together form the group of glycoprotein hormones which are formed in the pituitary and are secreted from there. Whereas the alpha subunit is common to the three hormones, their specificity of action is determined by the beta chain which is unique in each case. The molecular weight of FSH including the sugar portion is about 30 kD.

FSH and the other glycoprotein hormones act specifically via their selectively expressed G protein-coupled receptor (GPCR). FSH stimulates, through binding to its receptor, the association thereof with a stimulating G protein (Gₛ) which is thereby stimulated to hydrolyse guanosine triphosphate (GTP) and to activate the membrane-associated adenylate cyclase. Cyclic adenosine monophosphate (cAMP) is accordingly an important and readily quantifiable secondary messenger substance of FSH (G. Vassart, L. Pardo, S. Costagliola, Trends Biochem. Sci. 2004, 29, 119-126).

The importance of FSH for male fertility is the subject of intensive research. It has been possible to show that FSH influences several processes of spermatogenesis such as the proliferation of spermatogonia, the antiapoptotic effect on spermatogonia and spermatocytes and the stimulation of sperm maturation including motility thereof.

The following arguments are also in favour of the FSH receptor as target for male fertility control:
1. The FSH receptor is exclusively expressed on Sertoli cells (high specificity).
2. Contraceptive vaccination against FSH beta chain or the FSH receptor induces infertility in male primates (N. R. Mougdal, M. Jeyakumar, H. N. Krishnamurthy, S. Sridhar, H. Krishnamurthy, F. Martin, Human Reproduction Update 1997, 3, 335-346).
3. Naturally occurring mutations in the FSH receptor or the FSH beta chain may lead to sub- or infertility in men (I. Huhtaniemi, Journal of Reproduction and Fertility 2000, 119, 173-186; L. C. Layman, P. G. McDonough, Molecular and Cellular Endocrinology 2000, 161, 9-17).
4. Neutralizing FSH antiserum has no effect on testis weight and testosterone production (V. Sriraman, A. J. Rao, Molecular and Cellular Endocrionology 2004, 224, 73-82). Adverse effects of FSH blockade on androgen production therefore appear unlikely.

In line with these arguments, FSH antagonists are expected to be suitable for spermatogenesis inhibition (prevention) in men. Moreover, a suitable FSH antagonist may just as well lead to infertility in women, because it suppresses follicle ripening and thus also ovulation. On the other hand, the skilled person expects advantages from non-peptidergic FSH agonists when used to promote fertility in women (stimulation of follicle ripening). There are no reports of experience on the use of FSH or FSH agonists in male infertility, but specific indications are also conceivable in this connection.

New findings demonstrate that there is also a direct effect of FSH on cells of bone metabolism. There are two fundamentally different cell types in bones: osteoclasts and osteoblasts. While osteoclasts play a central role in bone resorption (breakdown of bone), osteoblasts simulate bone density (anabolic effect).

FSH receptors have been detected in osteoclasts but not in osteoblasts. In vitro, FSH stimulates bone resorption by mouse osteoclasts ( Li Sun et al. Cell 2006; 125: 247-60). A clinical correlation between the serum FSH level and low bone density has been observed in postmenopausal women (Devleta et al, J. Bone Miner. Metab. 2004, 22: 360-4).

These findings among others suggest that FSH stimulates loss of bone mass, and consequently FSH antagonists will display an antiresorptive effect on bone and are therefore suitable for the therapy and/or prevention of peri- and postmenopausal loss of bone mass and osteoporosis.

FSH receptor modulators are compounds that have a mixed profile of both FSH receptor antagonistic and FSH receptor agonistic properties. FSH receptor modulators of various compound classes of low molecular weight, have been reported on recently. FSH receptor modulators are disclosed in WO 2004/056779, WO 2004/056780; J. Med. Chem. 2005, 48, 1697 [tetrahydroquinolines]; WO 02/70493, Bioorg. Med. Chem. Lett. 2004, 14, 1713 and 1717 [diketopiperazines]; and WO 01/47875 [sulphonamides].

FSH receptor agonists are disclosed in WO 02/09706; J. Comb. Chem. 2004, 6, 196 [Thiazolidinones]; WO 2003/020726 and WO 03/20727, Chem. Biochem. 2002, 10, 1023 {thieno[2,3-d] pyrimidines)}; WO 01/87287 [pyrazoles]; WO 00/08015 [carbazoles]; WO 06/117023, WO 06/117368, WO 06/117370, WO 06/117371, [hexahydroquino-lines].

FSH receptor antagonists are disclosed in WO 03/004028 [tetrahydroquinolines], WO 02/09705 [thiazolidinones], WO 00/58277, Bioorg. Med. Chem. 2002, 10, 639 [sulphonic acids]; WO 00/58276, Endocr. 2002, 143, 3822; Synth. Comm. 2002, 32, 2695 [azo compounds]; US 2006/0199806, US 2006/0258644, US 2006/0258645, US 2006/0287522 [pyrrolobenzodiazepines], WO 2007/017289 [acyltryptophanols], EP06090223.6 [1 ,2-diarylacetylene derivatives of acyltryptophanols] and EP06077263.9 [bicyclic acyltryptophanols].

WO 2007/017289 is considered to be the closest prior art.

In view of the prior art, the objective technical problem to be solved according to the present invention may therefore be seen in providing alternative compounds having a FSH receptor antagonistic activity.

The technical problem has been solved according to the present invention by the provision of novel compounds of the formula I 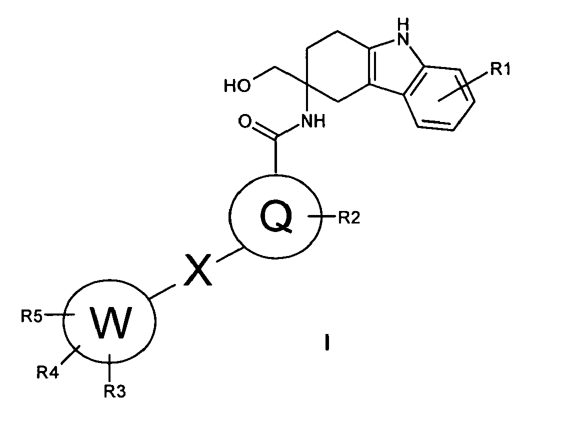 in which
- R1: is hydrogen, halogen, cyano, -SO₂Me, carboxy, -SO₂NH₂, -SO₃H, nitro, amino, C₁-C₆-alkyl, C₂-C₆-alkenyl, C₂-C₆-alkynyl or C₁-C₆-alkyloxy, where the hydrocarbon chains therein may optionally be substituted one or more times by fluorine, cyano or hydroxy;
- R2: is hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkylamino-C₁-C₆-alkylene, di(C₁-C₆-alkyl)amino-C₁-C₆-alkylene;
where the hydrocarbon chains therein may optionally be substituted one or more times by fluorine, cyano, hydroxy, amino or by the groups: 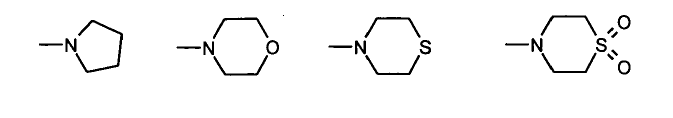 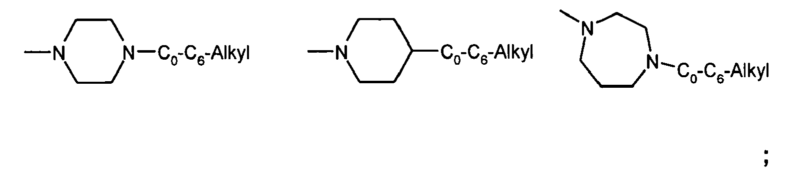
- R3, R4, R5: are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or by the radicals: 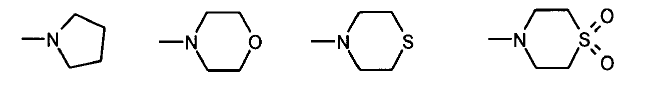 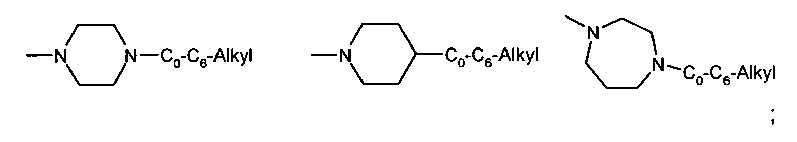 or
- R3, R4, R5: independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cydoalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene, C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino, C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneamino-carbonyl, C₁-C₆-alkylcarbonyl, C₃-C₇-cycloalkylcarbonyl, carboxy, carboxamido [-C(O)NH₂], C₁-C₆-alkyloxycarbonyl, C₁-C₃-alkylsulphanyl, C₁-C₆-alkysulphonyl, C₃-C₇-cydoalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl, -N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl, -C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine, -S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine, -O-C₂-C₆-alkylene-(C₁-C₆-alkyl)amine, -O-C₂-C₆-alkylene-[di(C₁-C₆-alkylene)]amine,
or the radicals: 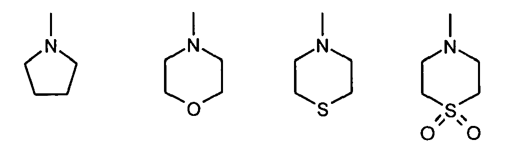 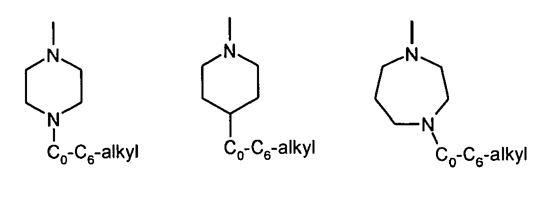 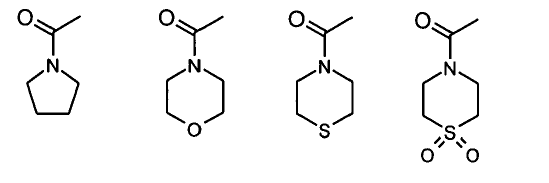 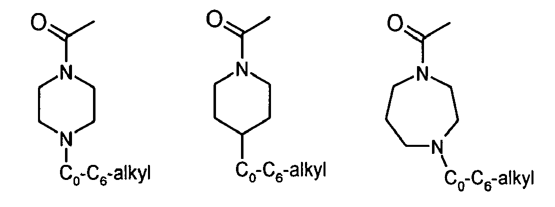 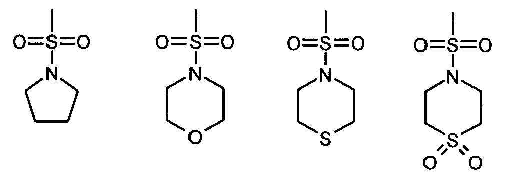 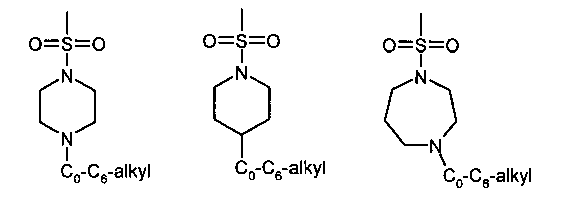 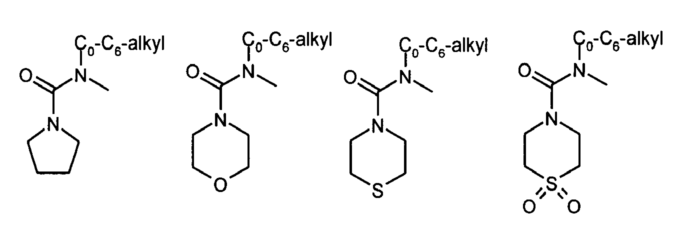 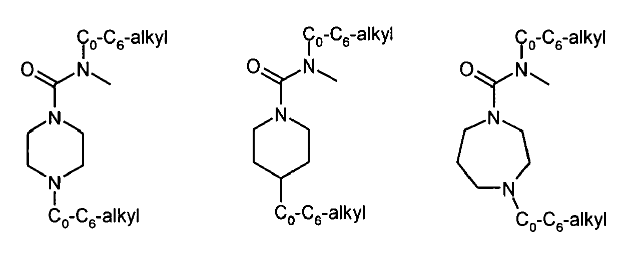 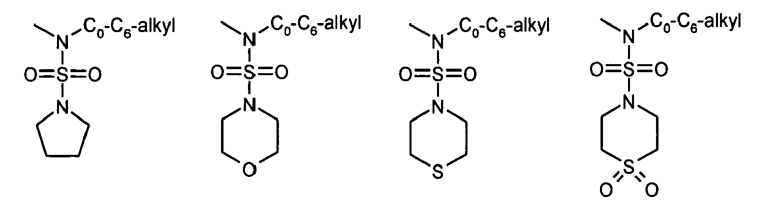 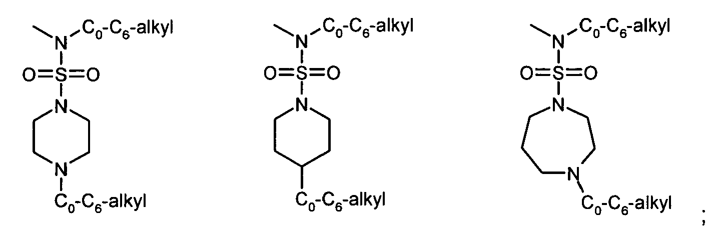
- R3 and R4: may together form heterocycloalkyl, cycloalkyl;
- Q: is an aryl or heteroaryl group
or the group 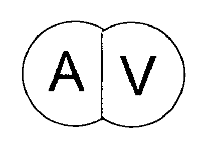 in which
A is a monocyclic aryl or a monocyclic heteroaryl group;
V is a cycloalkylen, cycloalkenylen, heterocycloalkylen or heterocycloalkenylen group;
- X: is a bond, C₁-C₄-alkylene, C₂-C₄-alkenylene, C₂-C₄-alkynylene;
- W: is an aryl or heteroaryl group;
where
- R1: may substitute one or more positions of the aryl ring in the tetrahydrocarbazole moiety;
- R2: may substitute one or more positions of the aryl or heteroaryl ring in the radical Q or in the radical V.

The present invention relates to both possible enantiomeric forms due to the stereo-centre of the tetrahydrocarbazole.

The unbranched C₁-C₆-alkyl groups for the radicals R1 to R5 may be for example a methyl, ethyl, propyl, butyl, pentyl or a hexyl group; and the branched C₃-C₆-alkyl groups for the radicals R1 to R6 may be an isopropyl, isobutyl, *sec*-butyl, *tert*-butyl, isopentyl, 2-methylbutyl, 1-methylbutyl, 1-ethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or a 1,2-dimethylbutyl group.
The branched or unbranched C₂-C₆-alkenyl groups for the radicals R1 to R5 may be for example a vinyl, allyl, (*E*)-2-methylvinyl, (*Z*)-2-methylvinyl, homoallyl, (*E*)-but-2-enyl, (Z)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (Z)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethyl-prop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (Z)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-l-enyl, (Z)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl- or a 1-(1,1-dimethylethyl)ethenyl group.

The C₂-C₆-alkynyl groups for the radicals R1 to R5 may be for example an ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

The C₁-C₆-alkyloxy groups for the radicals R1 to R5 may be for example a methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, neopentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1 ,3-dimethylbutyl)oxy or a (1,2-dimethylbutyl)oxy group.

The halogens for the radicals R1 to R5 include fluorine, chlorine, bromine or iodine.

The C₁-C₃-alkylsulphanyl groups for the radicals R3 to R5 may be for example a methylsulphanyl (CH₃S-), ethylsulphanyl (CH₃CH₂S-), propylsulphanyl, isopropyl-sulphanyl group.

The C₁-C₆-alkylaminocarbonyl groups for the radicals R3 to R5 may be for example a methylaminocarbonyl-, ethylaminocarbonyl-, propylaminocarbonyl-, isopropylaminocarbonyl-, butylaminocarbonyl-, isobutylaminocarbonyl-, sec-butylaminocarbonyl-, *tert-*butylaminocarbonyl-, pentylaminocarbonyl-, isopentylaminocarbonyl-, (2-methylbutyl)-aminocarbonyl-, (1-methylbutyl)aminocarbonyl-, (1-ethylpropyl)aminocarbonyl-, neo-pentylaminocarbonyl-, (1,1-dimethylpropyl)aminocarbonyl-, hexylaminocarbonyl-, (4-methylpentyl)aminocarbonyl-, (3-methylpentyl)aminocarbonyl-, (2-methylpentyl)aminocarbonyl-, (1-methylpentyl)aminocarbonyl-, (1-ethylbutyl)aminocarbonyl-, (2-ethylbutyl)-aminocarbonyl-, (3,3-dimethylbutyl)aminocarbonyl-, (2,2-dimethylbutyl)aminocarbonyl-, (1,1-dimethylbutyl)aminocarbonyl-, (2,3-dimethylbutyl)aminocarbonyl-, (1,3-dimethylbutyl)aminocarbonyl- or a (1,2-dimethylbutyl)aminocarbonyl group.

The hydroxy-C₁-C₆-alkylene groups for the radicals R2 to R5 may be a hydroxymethyl (HOCH₂-), 2-hydroxyethyl (HOCH₂CH₂-), 1-hydroxyethyl [CH₃CH(OH)-], 3-hydroxypropyl (HOCH₂CH₂CH₂-), 2-hydroxypropyl [CH₃CH(OH)CH₂-], 1-hydroxypropyl [CH₃CH₂CH(OH)-], 2-hydroxy-1-methylethyl [HOCH₂CH(CH₃)-], 1-hydroxy-1-methylethyl [(CH₃)₂C(OH)-], 4-hydroxybutyl (HOCH₂CH₂CH₂CH₂-), 3-hydroxybutyl [CH₃CH(OH)CH₂CH₂-], 2-hydroxybutyl [CH₃CH₂CH(OH)CH₂-], 1-hydroxybutyl [CH₃CH₂CH₂CH(OH)-], 3-hydroxy-1-methylpropyl [HOCH₂CH₂CH(CH₃)-], 2-hydroxy-1-methylpropyl [CH₃CH(OH)CH(CH₃)-], 1-hydroxy-1-methylpropyl [CH₃CH₂C(CH₃)(OH)-], 1-(hydroxymethyl)propyl [CH₃CH(CH₂OH)-], 3-hydroxy-2-methylpropyl [HOCH₂CH(CH₃)CH₂-], 2-hydroxy-2-methylpropyl [(CH₃)₂C(OH)CH₂-], 1-hydroxy-2-methylpropyl [CH₃CH(CH₃)CH(OH)-] or a 2-hydroxy-1,1-dimethylethyl group [HOCH₂C(CH₃)₂-].

The heterocycloalkyl groups which may form the radicals R3 and R4 together may be for example the following groups:

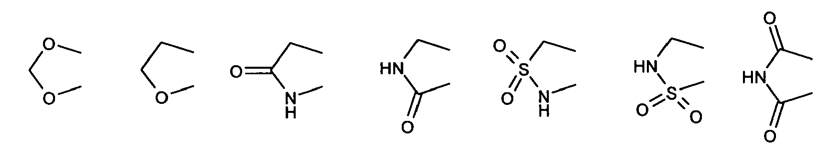

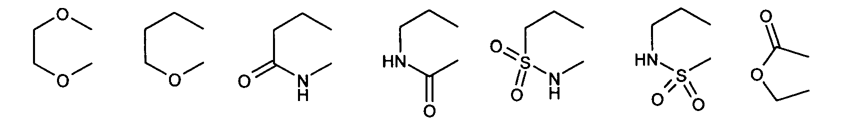

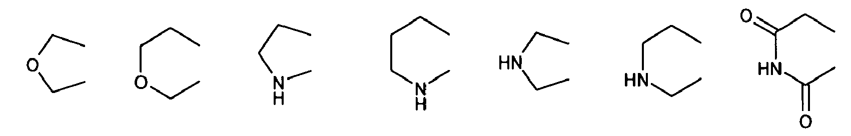

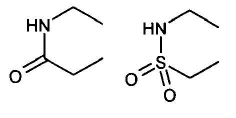

The cycloalkyl groups which may form the radicals R3 and R4 together may be for example the following groups:

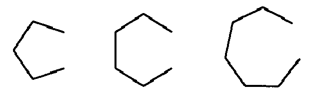

The C₃-C₇-cydoalkyl groups for the radicals R2 to R5 may be for example a cyclopropyl, cyclobutyl, cyclopentyl, cyclohexyl, cycloheptyl group.

The C₃-C₇-heterocydoalkyl groups for the radicals R3 to R5 may be for example a cyclopropyl, cyclobutyl, cycopentyl, cyclohexyl, cycloheptyl group in which one or two carbon atoms of the ring are replaced independently of one another by an oxygen, nitrogen or sulphur atom.

The monocyclic aryl group for A or Y may be for example a phenyl group which is linked via substitutable positions.

The aryl group for W or Q may be for example a phenyl, naphthyl group which is linked via substitutable positions.

The monocylic heteroaryl group for A or Y may be for example a pyridinyl, pyrimidinyl, furanyl, thienyl, oxazolyl, isoxazolyl, thiazolyl, pyrrolyl, pyrazolyl or an imidazolyl group which is linked via substitutable positions.

The heteroaryl group for W or Q may be for example a pyridinyl, pyrimidinyl, quinolinyl, isoquinolinyl, quinazolinyl, quinoxalinyl, phthalazinyl, 1.5-naphthyridinyl, 1,6-naphthyridinyl, 1,7-naphthyridinyl, 1,8-naphthyridinyl, benzofuranyl, benzothienyl, 1,3-benzodioxolyl, 2,1,3-benzothiadiazolyl, indolyl, indazolyl, furanyl, thienyl, oxazolyl, isoxazolyl, thiazolyl, pyrrolyl, pyrazolyl or an imidazolyl group which is linked via substitutable positions.

The heterocycloalkylen groups for V or Y may be for example the following groups:

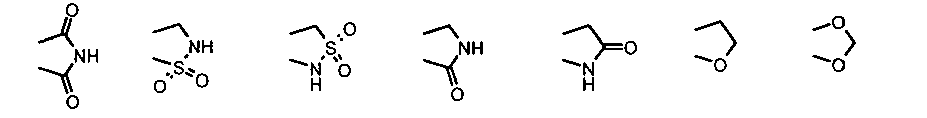

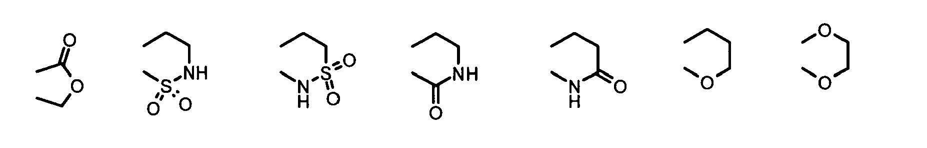

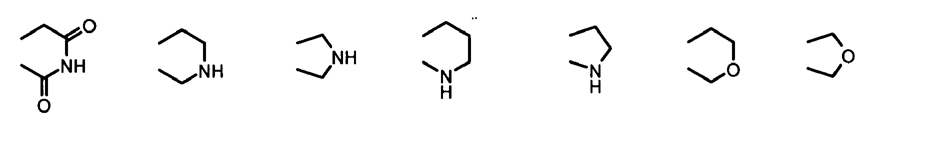

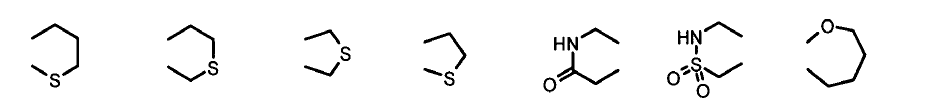

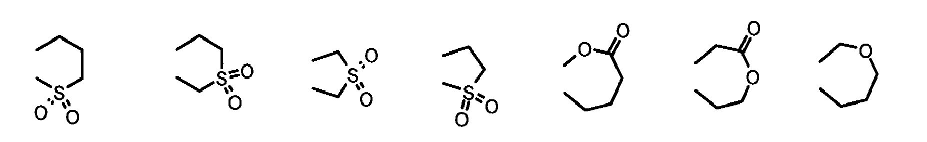

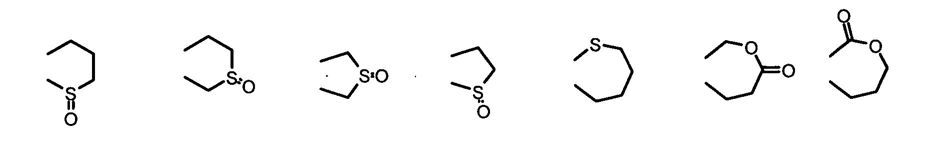

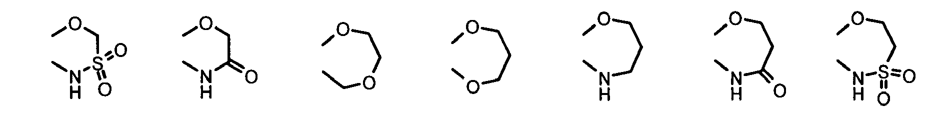

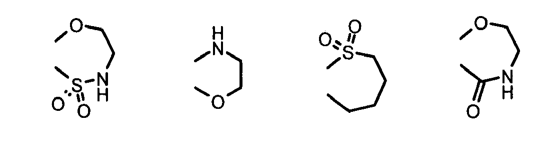

The heterocycloalkenylen groups for V or Y (see formulae III, V) may be for example the following groups:

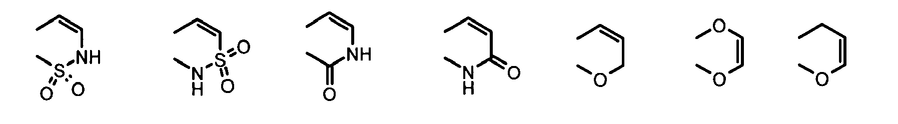

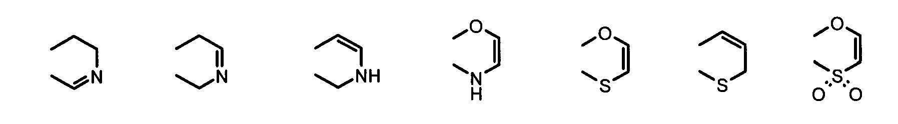

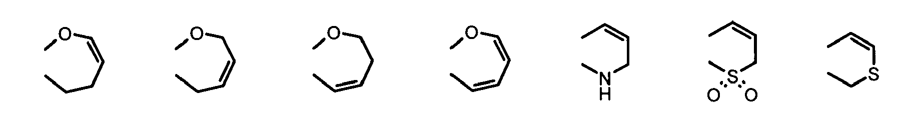

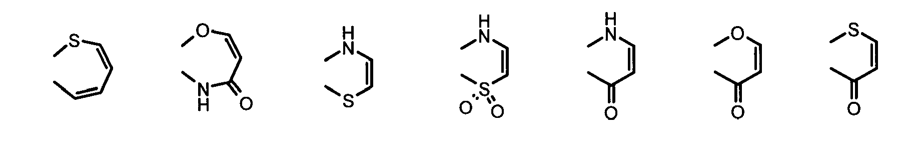

The cycloalkylen groups for V or Y may be for example the following groups:

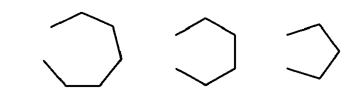

The cycloalkenylen groups for V or Y may be for example the following groups:

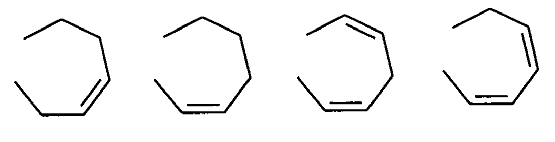

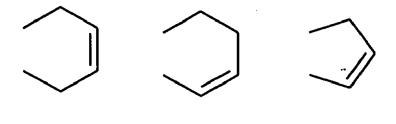

The C₁-C₄-alkylene groups for the radicals X may be for example a methylene (-CH₂-), ethylidene [-CH(CH₃)-], ethylene (-CH₂CH₂-), prop-1,3-ylene (-CH₂CH₂CH₂-), prop-1,2-ylene [-CH₂CH(CH₃)-], but-1,4-ylene (-CH₂CH₂CH₂CH₂-), but-1,3-ylene [-CH₂CH₂CH(CH₃)-], but-1,2-ylene [-CH₂CH(CH₂CH₃)-], but-2,3-ylene [-CHCH(CH₃)-], 2-methylprop-1,2-ylene [-CH₂C(CH₃)₂-] or a 2-methylprop-1 ,3-ylene group [-CH₂CH(CH₃)CH₂-].

The C₂-C₄-alkenylene groups for the radical X may be for example an ethen-1,2-ylidene (-CH=CH-), prop-2-en-1,3-ylidene (-CH₂-CH=CH-), prop-1-en-1,3-ylidene (-CH=CH-CH₂-), but-1-en-1,4-ylidene (-CH=CH-CH₂-CH₂-), but-2-en-1,4-ylidene (-CH₂-CH=CH-CH₂-) or a but-3-en-1,4-ylidene group (-CH₂-CH₂-CH=CH-).

The C₂-C₄-alkynylene groups for the radical X may be for example an ethyn-1,2-ylidene (-C≡C-), prop-2-yn-1,3-ylidene (-CH₂-C≡C-), prop-1-yn-1,3-ylidene (-C≡C-CH₂-), but-1-yn-1,4-ylidene (-C≡C-CH₂-CH₂-), but-2-yn-1,4-ylidene (-CH₂-C≡C-CH₂-) or a but-3-yn-1,4-ylidene group (-CH₂-CH₂-C≡C-).

The C₃-C₇-cycloalkyloxy groups for the radicals R2 to R5 may be for example a cyclo-propyloxy, cyclobutyloxy, cyclopentyloxy, cyclohexyloxy, cycloheptyloxy group.

The C₁-C₆-alkylamino groups for the radicals R2 to R5 may be for example methyl-amino, ethylamino, propylamino, isopropylamino, butylamino, isobutylamino, sec-butylamino, *tert*-butylamino, pentylamino, isopentylamino, (2-methylbutyl)amino, (1-methylbutyl)amino, (1-ethylpropyl)amino, neopentylamino, (1,1-dimethylpropyl)amino, hexyl-amino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1 ,2-dimethylbutyl)amino group.

In the di(C₁-C₆-alkyl)amino groups for the radicals R2 to R5, each of the two radicals on the nitrogen atom of the dialkylamino group may be chosen independently of one another from the following radicals: possible examples are a methyl, ethyl, propyl, isopropyl, butyl, *iso*butyl, *sec*-butyl, *tert*-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the C₃-C₇-cycloalkyl-C₁-C₆-alkyleneoxy groups for the radicals R2 to R5 it is possible to combine each of the C₃-C₇-cycloalkyl groups of the C₃-C₇-cycloalkyl-C₁-C₆-alkyleneoxy group, for example of a cyclopropyl, cyclobutyl, cyclopentyl, cyclohexyl or cycloheptyl group, independently of one another with each C0-C6-alkyleneoxy group, for example with a methyleneoxy, ethyleneoxy, propyleneoxy, butyleneoxy, pentylene-oxy, hexyleneoxy group.

In the hydroxy-C₃-C₆-alkenylene groups for the radicals R2 to R5 it is possible for the hydroxy group to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-Pent-2-enyl-, (*Z*)-Pent-2-enyl-, (*E*)-Pent-1-enyl-, (*Z*)-Pent-1-enyl-, hex-5-enyl-, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, ispropenyl 2-methylprop-2-enyl 1-methylprop-2-enyl, 2-methylprop-1-enyl, (*E*)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethyl-prop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (Z)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methylpent-1-enyl, (*E*)-2-methylpent-1-enyl, (*Z*)-2-methylpent-1-enyl, (*E*)-1-methytpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group, and to be combined independently of one another.

In the hydroxy-C₃-C₆-alkynyl groups for the radicals R2 to R5 it is possible for the hydroxy group to be located at any desired position of the C₃-C₆-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.

In the C₁-C₆-alkyloxy-C₃-C₆-alkenylene groups for the radicals R2 to R5 it is possible for the C₁-C₆-alkyloxy group, for example a methyloxy, ethyloxy, propyloxy, *iso*propyloxy, butyloxy, *iso*butyloxy, sec-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enuyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (Z)-4-methylpent-2-enyl, (*E*)-3-methylpent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methyl pent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group and to be combined independently of one another.

In the C₁-C₆-alkyloxy-C₃-C₆-alkynylene groups for the radicals R2 to R5 it is possible for the C₁-C₆-alkyloxy group, for example a methyloxy, ethyloxy, propyloxy, isopropyloxy, butyloxy, isobutyloxy, sec-butyloxy, *tert*-butyloxy, pentyloxy, isopentyloxy, (2-methylbutyl)oxy, (1-methylbutyl)oxy, (1-ethylpropyl)oxy, neopentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1,3-dimethylbutyl)oxy or a (1,2-dimethylbutyl)oxy group, to be located at any desired position of the C3-C6-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methyl-but-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-di-methylbut-3-ynyl, 1,1-dimethylbut-3-ynyl, 1,1-dimethylbut-2-ynyl or a 3,3-dimethylbut-1-ynyl group, and to be combined independently of one another.

In the C₁-C₆-alkyloxyphenyl-C₁-C₆-alkylene groups for the radical R3 to R5 it is possible for the C₁-C₆-alkyloxy group to be selected independently of one another from methyloxy, ethyloxy, propyloxy, isopropyloxy, butyloxy, isobutyloxy, sec-butyloxy, *tert*-butyl-oxy, pentyloxy, isopentyloxy, (2-methylbutyl)oxy, (1-methylbutyl)oxy, (1-ethylpropyl)oxy, neopentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy, and to be combined independently of one another with C₁-C₆-alkylene groups such as, for example, methylene, ethylene, propylene, butylene, pentylene, hexylene.

In the C₃-C₇-cycloalkyl-(C₀-C₆)-alkyleneamino groups of the radicals R3 to R5 it is possible for each of the C₃-C₇-cycloalkyl groups of the C₃-C₇-cycloalkyl-(C₀-C₆)-alkylene-amino group, for example of a cyclopropyl, cyclobutyl, cyclopentyl, cyclohexyl or cycloheptyl group, to be combined independently of one another with each C₀-C₆-alkylene group, for example with a bond, a methylene, ethylene, propylene, butylene, pentylene, hexylene group.

In the C₁-C₆-alkyloxy-C₁-C₆-alkylene groups for the radical R2 to R5, it is possible for the C₁-C₆-alkyloxy group to be selected independently for example from methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, neopentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy and to be combined independently of one another with C₁-C₆-alkylene groups such as, for example, methylene, ethylene, propylene, butylene, pentylene, hexylene.

In the di(C₁-C₆-alkyl)amino-C₁-C₆-alkylene group for the radicals R2 to R5 it is possible for each of the two radicals on the nitrogen atom of the amino group to be selected independently for example from methyl, ethyl, propyl, isopropyl, butyl, isobutyl, *sec*-butyl, *tert*-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1 ,2-dimethylbutyl) group, and to be combined with C₁-C₆-alkylene groups such as, for example, methylene, ethylene, propylene, butylene, pentylene, hexylene.

The C₃-C₇-cycloalkyl-C₁-C₆-alkylene groups for the radicals R2 to R5 may be for example a cyclopropyloxymethylene, cyclopropyloxyethylene, cyclopropyloxypropylene, cyclopropyloxybutylene, cyclopropyloxypentylene, cyclopropyloxyhexylene, cyclobutyloxymethylene, cyclobutyloxyethylene, cyclobutyloxypropylene, cyclobutyloxybutylene, cyclobutyloxypentylene, cyclobutyloxyhexylene, cyclopentyloxymethylene, cyclopentyloxyethylene, cyclopentyloxypropylene, cyclopentyloxybutylene, cyclopentyloxypentylene, cyclopentyloxyhexylene, cyclohexyloxymethylene, cyclohexyloxyethylene, cyclohexyloxypropylene, cyclohexyloxybutylene, cyclohexyloxypentylene, cyclohexyloxyhexylene, cycloheptyloxymethylene, cycloheptyloxyethylene, cycloheptyloxypropylene, cycloheptyloxybutylene, cycloheptyloxypentylene, cycloheptyloxyhexylen group.

In the C₁-C₆-alkylamino-C₁-C₆-alkylene groups for the radicals R2 to R5 it is possible for the C₁-C₆-alkylamino group to be selected independently for example from methyl-amino, ethylamino, propylamino, *iso*propylamino, butylamino, *iso*butylamino, *sec*-butylamino, *tert*-butylamino, pentylamino, *iso*pentylamino, (2-methylbutyl)amino, (1-methylbutyl)amino, (1-ethylpropyl)amino, *neo*pentylamino, (1,1-dimethylpropyl)amino, hexyl-amino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino and to be combined with C₁-C₆-alkylene groups such as, for example, methylene, ethylene, propylene, butylene, pentylene, hexylene.

The phenyloxy-C₁-C₆-alkylene groups for the radicals R3 to R5 may be for example a phenyloxymethyl, phenyloxyethyl, phenyloxypropyl, phenyloxybutyl, phenyloxypentyl, phenyloxyhexyl group.

In the C₁-C₆-acyl-(C₀-C₆-alkyl)amido groups for the radicals R3 to R5, it is possible for each of the C₁-C₆-acyl groups, for example a formyl, acetyl, propionyl, 2-methylpropionyl, 2,2-dimethylpropionyl, butyryl, 2-methylbutyryl, 3-methylbutyryl, 2,2-dimethyl-butyryl, 2-ethylbutyryl, pentanoyl, 2-methylpentanoyl, 3-methylpentanoyl, 4-methylpentanoyl or a hexanoyl group, to be combined independently of one another with each (C₀-C₆-alkyl)amido group, for example a hydrogen atom, a methylamido, ethylamido, propylamido, isopropylamido, butylamido, isobutylamido, sec-butylamido, tert-butylamido, pentylamido, isopentylamido, (2-methylbutyl)amido, (1-methylbutyl)amido, (1-ethylpropyl)amido, neopentylamido, (1,1-dimethylpropyl)amido, hexylamido, (4-methylpentyl)amido, (3-methylpentyl)amido, (2-methylpentyl)amido, (1-methylpentyl)amido, (1-ethylbutyl)amido, (2-ethylbutyl)amido, (3,3-dimethylbutyl)amido, (2,2-dimethylbutyl)-amido, (1,1-dimethylbutyl)amido, (2,3-dimethylbutyl)amido, (1,3-dimethylbutyl)amido or a (1,2-dimethylbutyl)amido group.

The C₁-C₆-alkylaminocarbonyl groups for the radicals R3 to R5 may be for example a methylaminocarbonyl, ethylaminocarbonyl, propylaminocarbonyl, isopropylaminocarbonyl, butylaminocarbonyl, isobutylaminocarbonyl, sec-butylaminocarbonyl, tert-butylaminocarbonyl, pentylaminocarbonyl, isopentylaminocarbonyl, (2-methylbutyl)-aminocarbonyl, (1-methylbutyl)aminocarbonyl, (1-ethylpropyl)aminocarbonyl, neopentylaminocarbonyl, (1,1-dimethylpropyl)aminocarbonyl, hexylaminocarbonyl, (4-methylpentyl)aminocarbonyl, (3-methylpentyl)aminocarbonyl, (2-methylpentyl)aminocarbonyl, (1-methylpentyl)aminocarbonyl, (1-ethylbutyl)aminocarbonyl, (2-ethylbutyl)aminocarbonyl, (3,3-dimethylbutyl)aminocarbonyl, (2,2-dimethylbutyl)-aminocarbonyl, (1,1-dimethylbutyl)aminocarbonyl, (2,3-dimethylbutyl)aminocarbonyl, (1,3-dimethylbutyl)aminocarbonyl or a (1,2-dimethylbutyl)aminocarbonyl group.

In the di(C₁-C₆-alkyl)aminocarbonyl groups for the radicals R3 to R5, each of the two C₁-C₆-alkyl radicals on the nitrogen atom of the di(C₁-C₆-alkyl)aminocarbonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminocarbonyl groups for the radicals R3 to R5 may be for example a cyclopropylaminocarbonyl, cyclobutylaminocarbonyl, cyclopentylaminocarbonyl, cyclohexylaminocarbonyl or cycloheptylaminocarbonyl group.

In the di(C₃-C₇-cycloalkyl)aminocarbonyl groups for the radicals R3 to R5, each of the two C₃-C₇-cycloalkyl radicals on the nitrogen atom of the di(C₃-C₇-cydoalkyl)aminocarbonyl group may be independently of one another for example a cyclopropyl, cyclobutyl, cyclopentyl, cyclohexyl or cycloheptyl group.

In the C₃-C₇-cycloalkyl-C₁-C₆-alkyleneaminocarbonyl groups of the radicals R3 to R5 it is possible for each of the C₃-C₇-cycloalkyl groups of the C₃-C₇-cycoalkyl-C₁-C₆-alkyleneaminocarbonyl groups, for example of a cyclopropyl, cyclobutyl, cyclopentyl, cyclohexyl or cycloheptyl group, to be combined independently of one another with each C₁-C₆-alkyleneaminocarbonyl group, for example with a methyleneaminocarbonyl, ethyleneaminocarbonyl, propyleneaminocarbonyl, butyleneaminocarbonyl, pentylene-aminocarbonyl, hexyleneaminocarbonyl group.

The C₁-C₆-alkylcarbonyl groups for the radicals R3 to R5 may be for example a methyl-carbonyl, ethylcarbonyl, propylcarbonyl, isopropylcarbonyl, butylcarbonyl, isobutylcar-bonyl, sec-butylcarbonyl, tert-butylcarbonyl, pentylcarbonyl, isopentylcarbonyl, (2-methylbutyl)carbonyl, (1-methylbutyl)carbonyl, (1-ethylpropyl)carbonyl, neopentylcar-bonyl, (1,1-dimethylpropyl)carbonyl, hexylcarbonyl, (4-methylpentyl)carbonyl, (3-methylpentyl)carbonyl, (2-methylpentyl)carbonyl, (1-methylpentyl)carbonyl, (1-ethylbutyl)-carbonyl, (2-ethylbutyl)carbonyl, (3,3-dimethylbutyl)carbonyl, (2,2-dimethylbutyl)carbonyl, (1,1-dimethylbutyl)carbonyl, (2,3-dimethylbutyl)carbonyl, (1,3-dimethylbutyl)-carbonyl or a (1,2-dimethylbutyl)carbonyl group.

The C₃-C₇-cycloalkylcarbonyl groups for the radicals R3 to R5 may be for example a cyclopropylcarbonyl, cyclobutylcarbonyl, cyclopentylcarbonyl, cyclohexylcarbonyl or cycloheptylcarbonyl group.

The C₁-C₆-alkyloxycarbonyl groups for the radicals R3 to R5 may be for example a methyloxycarbonyl, ethyloxycarbonyl, propyloxycarbonyl, isopropyloxycarbonyl, butyloxycarbonyl, isobutyloxycarbonyl, sec-butyloxycarbonyl, tert-butyloxycarbonyl, pentyloxycarbonyl, isopentyloxycarbonyl, (2-methylbutyl)oxycarbonyl, (1-methylbutyl)oxycarbonyl, (1-ethylpropyl)oxycarbonyl, neopentyloxycarbonyl, (1,1-dimethylpropyl)oxycarbonyl, hexyloxycarbonyl, (4-methylpentyl)oxycarbonyl, (3-methylpentyl)oxycarbonyl, (2-methylpentyl)oxycarbonyl, (1-methylpentyl)oxycarbonyl, (1-ethylbutyl)oxycarbonyl, (2-ethylbutyl)oxycarbonyl, (3,3-dimethylbutyl)oxycarbonyl, (2,2-dimethylbutyl)oxycarbonyl, (1,1-dimethylbutyl)oxycarbonyl, (2,3-dimethylbutyl)oxycarbonyl, (1,3-dimethylbutyl)oxycarbonyl or a (1,2-dimethylbutyl)oxycarbonyl group.

The C₁-C₆-alkylsulphonyl groups for the radicals R3 to R5 may be for example a methylsulphonyl, ethylsulphonyl, propylsulphonyl, isopropylsulphonyl, butylsulphonyl, isobutylsulphonyl, sec-butylsulphonyl, tert-butylsulphonyl, pentylsulphonyl, isopentylsulphonyl, (2-methylbutyl)sulphonyl, (1-methylbutyl)sulphonyl, (1-ethylpropyl)sulphonyl, neopentylsulphonyl, (1,1-dimethylpropyl)sulphonyl, hexylsulphonyl, (4-methylpentyl)-sulphonyl, (3-methylpentyl)sulphonyl, (2-methylpentyl)sulphonyl, (1-methylpentyl)-sulphonyl, (1-ethylbutyl)sulphonyl, (2-ethylbutyl)sulphonyl, (3,3-dimethylbutyl)sulphonyl, (2,2-dimethylbutyl)sulphonyl, (1,1-dimethylbutyl)sulphonyl, (2,3-dimethylbutyl)sulphonyl, (1 ,3-dimethylbutyl)sulphonyl or a (1,2-dimethylbutyl)sulphonyl group.

The C₃-C₇-cycloalkylsulphonyl groups for the radicals R3 to R5 may be for example a cyclopropylsulphonyl, cyclobutylsulphonyl, cyclopentylsulphonyl, cyclohexylsulphonyl or cycloheptylsulphonyl group.

In the C₃-C₇-cycloalkyl-C₁-C₆-alkylenesulphonyl groups of the radicals R3 to R5 it is possible for each of the C₃-C₇-cycloalkyl groups of the C₃-C₇-cycloalkyl-C₁-C₆-alkylene-sulphonyl groups, for example of a cyclopropyl, cyclobutyl, cyclopentyl, cyclohexyl or cycloheptyl group, to be combined independently of one another with each C₁-C₆-alkylenesulphonyl group, for example with a methylenesulphonyl, ethylenesulphonyl, propylenesulphonyl, butylenesulphonyl, pentylenesulphonyl, hexylenesulphonyl group. The C₁-C₆-alkylaminosulphonyl groups for the radicals R3 to R5 may be for example a methylaminosulphonyl, ethylaminosulphonyl, propylaminosulphonyl, isopropylaminosulphonyl, butylaminosulphonyl, isobutylaminosulphonyl, sec-butylaminosulphonyl, tert-butylaminosulphonyl, pentylaminosulphonyl, isopentylaminosulphonyl, (2-methylbutyl)aminosulphonyl, (1-methylbutyl)aminosulphonyl, (1-ethylpropyl)aminosulphonyl, neopentylaminosulphonyl, (1,1-dimethylpropyl)aminosulphonyl, hexylaminosulphonyl, (4-methylpentyl)aminosulphonyl, (3-methylpentyl)aminosulphonyl, (2-methylpentyl)-aminosulphonyl, (1-methylpentyl)aminosulphonyl, (1-ethylbutyl)aminosulphonyl, (2-ethylbutyl)aminosulphonyl, (3,3-dimethylbutyl)aminosulphonyl, (2,2-dimethylbutyl)-aminosulphonyl, (1,1-dimethylbutyl)aminosulphonyl, (2,3-dimethylbutyl)aminosulphonyl, (1,3-dimethylbutyl)aminosulphonyl or a (1,2-dimethylbutyl)aminosulphonyl group.

In the di(C₁-C₆-alkyl)aminosulphonyl groups for the radicals R4 to R6, each of the two C₁-C₆-alkyl radicals on the nitrogen atom of the di(C₁-C₆-alkyl)aminosulphonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

The (C₃-C₇-cycloalkyl)aminosulphonyl groups for the radicals R3 to R5 may be for example a cyclopropylaminosulphonyl, cyclobutylaminosulphonyl, cyclopentylaminosulphonyl, cyclohexylaminosulphonyl or cycloheptylaminosulphonyl group.

In the di(C₃-C₇-cycloalkyl)aminosulphonyl groups for the radicals R3 to R5, each of the two C₃-C₇-cycloalkyl radicals on the nitrogen atom of the di(C₃-C₇-cycloalkyl)amino-sulphonyl group may be independently of one another for example a cyclopropyl, cyclobutyl, cyclopentyl, cyclohexyl or cycloheptyl group.

In the C₃-C₇-cydoalkyl-C₁-C₆-alkyleneaminosulphonyl groups of the radicals R3 to R5, each of the C₃-C₇-cycloalkyl groups of the C₃-C₇-cydoalkyl-C₁-C₆-alkyleneamino-sulphonyl groups, for example of a cyclopropyl, cyclobutyl, cyclopentyl, cyclohexyl or cycloheptyl group, can be combined independently of one another with each C₁-C₆-alkyleneaminosulphonyl group, for example with a methyleneaminosulphonyl, ethyleneaminosulphonyl, propyleneaminosulphonyl, butyleneaminosulphonyl, pentyleneaminosulphonyl, hexyleneaminosulphonyl group.

The C₁-C₆-alkylsulphonylamido groups for the radicals R3 to R5 may be for example a methylsulphonylamido, ethylsulphonylamido, propylsulphonylamido, isopropylsulphonylamido, butylsulphonylamido, isobutylsulphonylamido, sec-butylsulphonylamido, tert-butylsulphonylamido, pentylsulphonylamido, isopentylsulphonylamido, (2-methylbutyl)-sulphonylamido, (1-methylbutyl)sulphonylamido, (1-ethylpropyl)sulphonylamido, neopentylsulphonylamido, (1,1-dimethylpropyl)sulphonylamido, hexylsulphonylamido, (4-methylpentyl)sulphonylamido, (3-methylpentyl)sulphonylamido, (2-methylpentyl)-sulphonylamido, (1-methylpentyl)sulphonylamido, (1-ethylbutyl)sulphonylamido, (2-ethylbutyl)sulphonylamido, (3,3-dimethylbutyl)sulphonylamido, (2,2-dimethylbutyl)-sulphonylamido, (1,1-dimethylbutyl)sulphonylamido, (2,3-dimethylbutyl)sulphonylamido, (1,3-dimethylbutyl)sulphonylamido or a (1,2-dimethylbutyl)sulphonylamido group.

In the -N(C₀-C₆-alkyl)-C(O)-C₁-C₆-alkyl groups of the radicals R3 to R5, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₁-C₆-alkyl group on the carbonyl group of the amide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1 ,2-dimethylbutyl) group.

In the -N-(C₀-C₆-alkyl)-C(O)-C₃-C₇-cycloalkyl groups of the radicals R3 to R5, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the carbonyl group of the amide, for example with a cyclopropyl, cyclobutyl, cyclopentyl, cyclohexyl or cycloheptyl group.

In the -N(C₀-C₆-alkyl)-C(O)-N-di(C₀-C₆-alkyl) groups of the radicals R3 to R5, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-O-(C₀-C₆-alkyl) groups of the radicals R3 to R5, both (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-NH-(C₃-C₇-cycloalkyl) groups of the radicals R3 to R5, each of the (C₀-C₆-alkyl) groups on the nitrogen atom of the -N(C₀-C₆-alkyl)-C(O)-NH-(C₃-C₇-cycloalkyl) groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₃-C₇-cycloalkyl group on the terminal nitrogen atom of the urea, for example with a cyclopropyl, cyclobutyl, cyclopentyl, cyclohexyl or cycloheptyl group.

In the -N(C₀-C₆-alkyl)-SO₂-(C₁-C₆-alkyl) groups of the radicals R3 to R5, each of the (C₀-C₆-alkyl) groups on the nitrogen atom of the -N(C₀-C₆-alkyl)-SO₂-(C₁-C₆-alkyl) group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₁-C₆-alkyl group on the sulphonyl group of the sulphonamide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-C₃-C₇-cycloalkyl groups of the radicals R3 to R5, each of the (C₀-C₆-alkyl) groups on the nitrogen atom of the -N(C₀-C₆-alkyl)-SO₂-C₃-C₇-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the sulphonyl group, for example with a cyclopropyl, cyclobutyl, cyclopentyl, cyclohexyl or cycloheptyl group.

In the -N(C₀-C₆-alkyl)-SO₂-N-di(C₀-C₆-alkyl) groups of the radicals R3 to R5, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-SO₂-NH-(C₃-C₇)-cycloalkyl groups of the radicals R3 to R5, the C₀-C₆-alkyl group of the -N(C₀-C₆-alkyl)-SO₂-NH-(C₃-C₇)-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group, for example with a cyclopropyl, cyclobutyl, cyclopentyl, cyclohexyl or cycloheptyl group.

In the -C(O)-N(H)-C₂-C₆-alkylene-(C₁-C₆-alkyl)amine groups of the radicals R3 to R5, each of the C₂-C₆-alkylene groups on the nitrogen atom of the -C(O)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -C(O)-N(H)-C₂-C₆-alkylene-[di(C₁-C₆-alkyl)]amine groups of the radicals R3 to R5, each of the C₂-C₆-alkylene groups on the nitrogen atom of the -C(O)-N(H)-C₂-C₆-alkylene-[di(C₁-C₆-alkyl)]amine group, for example an ethylene, propylene, butylene, pentylene or hexylene group, may be combined independently of one another with each of the two identically or different C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1 ,2-dimethylbutyl) group.

In the -C(O)-N(H)-C₂-C₆-alkylene-(C₃-C₇-cycloalkyl)amine groups of the radicals R3 to R5, each of the (C₂-C₆-alkylene) groups of the -C(O)-N(H)-C₂-C₆-alkylene-(C₃-C₇-cycloalkyl)amine group, for example an ethylene, propylene, butylene, pentylene or hexylene group, may be combined independently of one another with each C₃-C₇-cycloalkyl group on the amine, for example with a cyclopropyl, cyclobutyl, cyclopentyl, cyclohexyl or cycloheptyl group.

In the -C(O)-N(H)-C₂-C₆-alkylene-(C₃-C₇-cycloalkyl-C₁-C₆-alkylene)amine groups of the radicals R3 to R5, each of the (C₂-C₆-alkylene) groups of the -C(O)-N(H)-C2-C6-alkylene-(C3-C6-cycloalkyl-C1-C6-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylene or cycloheptylhexylene group.

In the -S(O₂)-N(H)-C₂-C₆-alkylene-(C₁-C₆-alkyl)amine groups of the radicals R3 to R5, the (C₂-C₆-alkylene) groups of the -S(O₂)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-[di(C₁-C₆-alkyl)]amine groups of the radicals R3 to R5, the C₂-C₆-alkylene group of the -S(O₂)-N(H)-C₂-C₆-alkylene-[di(C₁-C₆-alkyl)]amine group, for example an ethylene, propylene, butylene, pentylene or hexylene group, may be combined independently of one another with each of the two C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-(C₃-C₇-cycloalkyl)amine groups of the radicals R3 to R5, the C₂-C₆-alkylene group of the -S(O₂)-N(H)-C₂-C₆-alkylene-(C₃-C₇-cycloalkyl)-amine group, for example an ethylene, propylene, butylene, pentylene or hexylene group, may be combined independently of one another with each C₃-C₇-cycloalkyl group on the amino group, for example with a cyclopropyl, cyclobutyl, cyclopentyl, cyclohexyl or cycloheptyl group.

In the -S(O₂)-N(H)-C₂-C₆-alkylene-(C₃-C₇-cycloalkyl-C₁-C₆-alkylene)amine groups of the radicals R3 to R5, each C₂-C₆-alkylene group of the -S(O₂)-N(H)-C₂-C₆-alkylene-(C₃-C₇-cydoalkyl-C₁-C₆-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylen or cycloheptylhexylene group.

In the -O-C₂-C₆-alkylene-(C₁-C₆-alkyl)amine groups of the radicals R3 to R5, the C₂-C₆-alkylene group of the -O-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -O-C₂-C₆-alkylene-[di(C₁-C₆-alkyl)]amine groups of the radicals R3 to R5, the C₂-C₆-alkylene group of the -O-C₂-C₆-alkylene-[di(C₁-C₆-alkyl)]amine group, for example an ethylene, propylene, butylene, pentylene or hexylene group, may be combined independently of one another with two freely selectable C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

Compounds preferred according to the present invention are those of the formula II 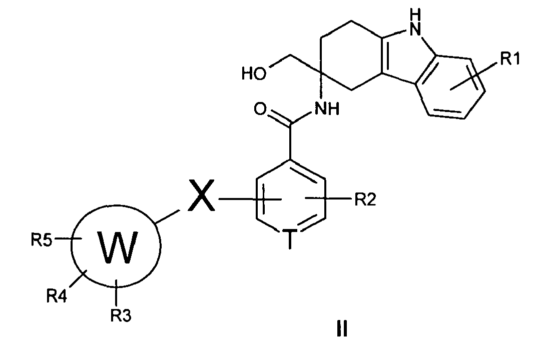 wherein
T is a nitrogen atom or a CH group; and
R1, R2, R3, R4, R5, X, and W have the same meaning as defined in formula I.

Compounds likewise preferred according to the present invention are those of the formula III 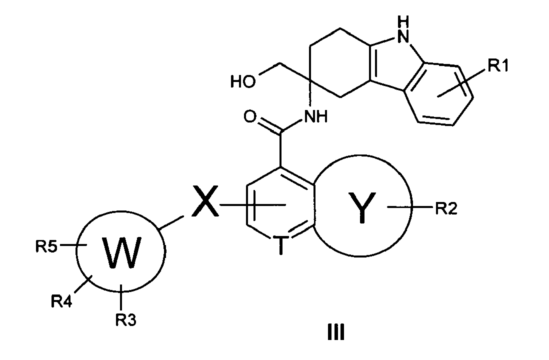 wherein
- T: is a nitrogen atom or a CH group;
- Y: is a monocyclic aryl or a monocyclic heteroaryl group or a cycloalkylen, cycloalkenylen, heterocycloalkylen or heterocycloalkenylen group;
and
- R1, R2,: R3, R4, R5, X and W have the same meaning as defined in formula I;
where
- R2: may substitute one or more positions of the aryl or heteroaryl ring in the radical Y.

Compounds particularly preferred according to the present invention are those of the formula IV 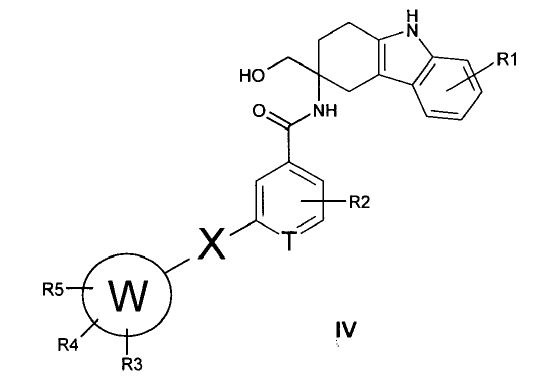 wherein
- T: is a nitrogen atom or a CH group; and
R1, R2, R3, R4, R5, X, and W have the same meaning as defined in formula I.

Compounds likewise particularly preferred according to the present invention are those of the formula V 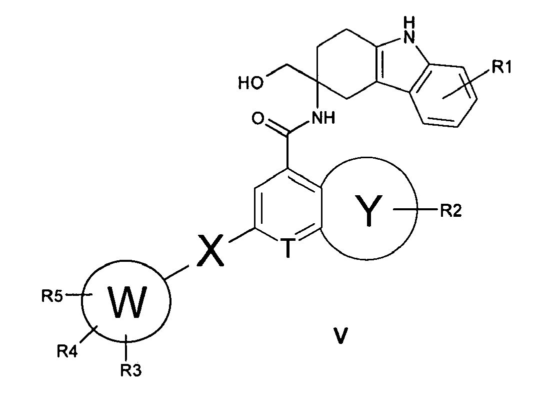 wherein
- T: is a nitrogen atom or a CH group;
- Y: is a monocyclic aryl or a monocyclic heteroaryl group or a cycloalkylen, cycloalkenylen, heterocycloalkylen or heterocycloalkenylen group;
and
R1, R2, R3, R4, R5, X and W have the same meaning as defined in formula I;
where
- R2: may substitute one or more positions of the aryl or heteroaryl ring in the radical Y.

The following compounds are most particularly preferred:
**1** 6-Methoxy-2-(3,4,5-trimethoxy-phenyl)-quinoline-4-carboxylic acid ((S)-3-hydroxymethyl-2,3,4,9-tetrahydro-1H-carbazol-3-yl)-amide
**2** 6-Methoxy-2-phenyl-quinoline-4-carboxylic acid (5,8-dichloro-3-hydroxymethyl-2,3,4,9-tetrahydro-1H-carbazol-3-yl)-amide
**3** 6-Methoxy-2-phenyl-quinoline-4-carboxylic acid (3-hydroxymethyl-8-trifluoromethyl-2,3,4,9-tetrahydro-1H-carbazol-3-yl)-amide
**4** 6-Methoxy-2-phenyl-quinoline-4-carboxylic acid (3-hydroxymethyl-2,3,4,9-tetrahydro-1H-carbazol-3-yl)-amide
**5** 6-Hydroxymethyl-6-[(6-methoxy-2-phenyl-quinoline-4-carbonyl)-amino]-6,7,8,9-tetrahydro-5H-carbazole-3-carboxylic acid
**6** 6-Methoxy-2-phenyl-quinoline-4-carboxylic acid (8-chloro-3-hydroxymethyl-2,3,4,9-tetrahydro-1H-carbazol-3-yl)-amide
**7** 6-Methoxy-2-phenyl-quinoline-4-carboxylic acid (5-chloro-3-hydroxymethyl-6-methyl-2,3,4,9-tetrahydro-1H-carbazol-3-yl)-amide
**8** 6-Methoxy-2-phenyl-quinoline-4-carboxylic acid (6-chloro-3-hydroxymethyl-8-methyl-2,3,4,9-tetrahydro-1H-carbazol-3-yl)-amide
**9** 6-Methoxy-2-phenyl-quinoline-4-carboxylic acid (3-hydroxymethyl-5,8-dimethyl-2,3,4,9-tetrahydro-1H-carbazol-3-yl)-amide
**10** 6-Methoxy-2-phenyl-quinoline-4-carboxylic acid (3-hydroxymethyl-6-trifluoromethyl-2,3,4,9-tetrahydro-1H-carbazol-3-yl)-amide
**11** 6-Methoxy-2-phenyl-quinoline-4-carboxylic acid (3-hydroxymethyl-6-trifluoromethoxy-2,3,4,9-tetrahydro-1H-carbazol-3-yl)-amide
**12** 6-Methoxy-2-phenyl-quinoline-4-carboxylic acid (8-fluoro-3-hydroxymethyl-2,3,4,9-tetrahydro-1H-carbazol-3-yl)-amide
**13** 6-Methoxy-2-phenyl-quinoline-4-carboxylic acid (7-chloro-6-fluoro-3-hydroxymethyl-2,3,4,9-tetrahydro-1H-carbazol-3-yl)-amide
**14** 6-Methoxy-2-phenyl-quinoline-4-carboxylic acid (3-hydroxymethyl-6-sulfamoyl-2,3,4,9-tetrahydro-1H-carbazol-3-yl)-amide
**15** 6-Methoxy-2-phenyl-quinoline-4-carboxylic acid (5,6-dichloro-3-hydroxymethyl-2,3,4,9-tetrahydro-1H-carbazol-3-yl)-amide
**16** 4-lsopropoxy-3',4'-dimethoxy-biphenyl-3-carboxylic acid (3-hydroxymethyl-2,3,4,9-tetrahydro-1H-carbazol-3-yl)-amide
**17** 4-lsopropoxy-3',4'-dimethoxy-biphenyl-3-carboxylic acid (8-chloro-3-hydroxymethyl-2,3,4,9-tetrahydro-1H-carbazol-3-yl)-amide
**18** 4-Isopropoxy-3',4'-dimethoxy-biphenyl-3-carboxylic acid (6-fluoro-3-hydroxymethyl-2,3,4,9-tetrahydro-1H-carbazol-3-yl)-amide
**19** 4-Isopropoxy-3',4'-dimethoxy-biphenyl-3-carboxylic acid (3-hydroxymethyl-5,8-dimethyl-2,3,4,9-tetrahydro-1H-carbazol-3-yl)-amide
**20** 4-Isopropoxy-3',4'-dimethoxy-biphenyl-3-carboxylic acid (3-hydroxymethyl-6-trifluoromethyl-2,3,4,9-tetrahydro-1H-carbazol-3-yl)-amide
**21** 4-Isopropoxy-3',4'-dimethoxy-biphenyl-3-carboxylic acid (3-hydroxymethyl-6-trifluoromethoxy-2,3,4,9-tetrahydro-1H-carbazol-3-yl)-amide
**22** 4-isopropoxy-3',4'-dimethoxy-biphenyl-3-carboxylic acid (8-fluoro-3-hydroxymethyl-2,3,4,9-tetrahydro-1H-carbazol-3-yl)-amide
**23** N-(3-Hydroxymethyl-8-trifluoromethyl-2,3,4,9-tetrahydro-1H-carbazol-3-yl)-2-isopropoxy-5-(2-methoxy-phenylethynyl)-benzamide
**24** N-(3-Hydroxymethyl-2,3,4,9-tetrahydro-1H-carbazol-3-yl)-2-isopropoxy-5-(2-methoxy-henylethynyl)-benzamide
**25** 3'-Chloro-4-isopropoxy-biphenyl-3,4'-dicarboxylic acid 3-[(5,8-dichloro-3-hydroxymethyl-2,3,4,9-tetrahydro-1H-carbazol-3-yl)-amide] 4'-methylamide
**26** 3'-Chloro-4-isopropoxy-biphenyl-3,4'-dicarboxylic acid 3-[(3-hydroxymethyl-8-trifluoromethyl-2,3,4,9-tetrahydro-1H-carbazol-3-yl)-amide] 4'-methylamide
**27** 3'-Chloro-4-isopropoxy-biphenyl-3,4'-dicarboxylic acid 3-[(3-hydroxymethyl-2,3,4,9-tetrahydro-1H-carbazol-3-yl)-amide]4'-methylamide
**28** 6-[(3'-Chloro-4-isopropoxy-4'-methylcarbamoyl-biphenyl-3-carbonyl)-amino]-6-hydroxymethyl-6,7,8,9-tetrahydro-5H-carbazole-1-carboxylic acid
**29** 6-[(3'-Chloro-4-isopropoxy-4'-methylcarbamoyl-biphenyl-3-carbonyl)-amino]-6-hydroxymethyl-6,7,8,9-tetrahydro-5H-carbazole-3-carboxylic acid
**30** 3'-Chloro-4-isopropoxy-biphenyl-3,4'-dicarboxylic acid 3-[(8-chloro-3-hydroxymethyl-2,3,4,9-tetrahydro-1H-carbazol-3-yl)-amide] 4'-methylamide
**31** 3'-Chloro-4-isopropoxy-biphenyl-3,4'-dicarboxylic acid 3-[(5-chloro-3-hydroxymethyl-6-methyl-2,3,4,9-tetrahydro-1H-carbazol-3-yl)-amide] 4'-methylamide
**32** 3'-Chloro-4-isopropoxy-biphenyl-3,4'-dicarboxylic acid 3-[(3-hydroxymethyl-6-methyl-2,3,4,9-tetrahydro-1 H-carbazol-3-yl)-amide] 4'-methylamide
**33** 3'-Chloro-4-isopropoxy-biphenyl-3,4'-dicarboxylic acid 3-[(6-fluoro-3-hydroxymethyl-2,3,4,9-tetrahydro-1H-carbazol-3-yl)-amide] 4'-methylamide
**34** 3'-Chloro-4-isopropoxy-biphenyl-3,4'-dicarboxylic acid 3-[(6-chloro-3-hydroxymethyl-2,3,4,9-tetrahydro-1H-carbazol-3-yl)-amide] 4'-methylamide
**35** 3'-Chloro-4-isopropoxy-biphenyl-3,4'-dicarboxylic acid 3-[(7-chloro-6-fluoro-3-hydroxymethyl-2,3,4,9-tetrahydro-1H-carbazol-3-yl)-amide] 4'-methylamide
**36** 5-Benzofuran-2-yl-N-(5,8-dichloro-3-hydroxymethyl-2,3,4,9-tetrahydro-1H-carbazol-3-yl)-2-isopropoxy-benzamide
**37** 5-Benzofuran-2-yl-N-(3-hydroxymethyl-8-trifluoromethyl-2,3,4,9-tetrahydro-1 H-carbazol-3-yl)-2-isopropoxy-benzamide
**38** 5-Benzofuran-2-yl-N-(3-hydroxymethyl-2,3,4,9-tetrahydro-1H-carbazol-3-yl)-2-isopropoxy-benzamide
**39** 6-(5-Benzofuran-2-yl-2-isopropoxy-benzoylamino)-6-hydroxymethyl-6,7,8,9-tetrahydro-5H-carbazole-3-carboxylic acid
**40** 5-Benzofuran-2-yl-N-(5-chloro-3-hydroxymethyl-6-methyl-2,3,4,9-tetrahydro-1 H-carbazol-3-yl)-2-isopropoxy-benzamide
**41** 5-Benzofuran-2-yl-N-(3-hydroxymethyl-6-methyl-2,3,4,9-tetrahydro-1H-carbazol-3-yl)-2-isopropoxy-benzamide
**42** 5-Benzofuran-2-yl-N-(6-fluoro-3-hydroxymethyl-2,3,4,9-tetrahydro-1 H-carbazol-3-yl)-2-isopropoxy-benzamide
**43** 5-Benzofuran-2-yl-N-(6-chloro-3-hydroxymethyl-8-methyl-2,3,4,9-tetrahydro-1 H-carbazol-3-yl)-2-isopropoxy-benzamide
**44** 5-Benzofuran-2-yl-N-(3-hydroxymethyl-5,8-dimethyl-2,3,4,9-tetrahydro-1 H-carbazol-3-yl)-2-isopropoxy-benzamide
**45** 5-Benzofuran-2-yl-N-(3-hydroxymethyl-5,7-dimethyl-2,3,4,9-tetrahydro-1H-carbazol-3-yl)-2-isopropoxy-benzamide
**46** 5-Benzofuran-2-yl-N-(3-hydroxymethyl-8-methyl-2,3,4,9-tetrahydro-1H-carbazol-3-yl)-2-isopropoxy-benzamide

### Pharmacological investigations

### HTRF assay for measuring cAMP in cells

The method is based on a competitive immunoassay between native cAMP, which has been produced by the cells, and cAMP which is labelled with XL665. The tracer binding was visualized by a monoclonal antibody, anti-cAMP labelled with cryptate [HTRF = homogeneous time-resolved fluorescence].

The specific signal is inversely proportional to the cAMP concentration of the samples employed.

The 665nm/ 620nm fluorescence ratio was evaluated.

*The following material was used: 96-well plates for the tissue culture, 96-well plates with black edge and black base (e.g. Fluotrac 600 from Greiner), 96-well plates for the substance dilutions of polypropylene and cAMP Femtomolar (4000wells Kit, CIS Bio International # 62AM1PEC).*

The following reagents were used: BSA (bovine serum albumin) Fraction V protease-free, IBMX (3-isobutyl-1-methylxanthine), hFSH (human follicle stimulating hormone), Triton X-100 analytical grade, potassium fluoride analytical grade, G 418 (Geneticin) and Accutase.

Buffer 1 (washing and testing buffer) contained PBS, 1 mM CaCl2, 1 mM MgCl₂, 0.2% glucose; 0.1% BSA, 1 mM IBMX.

Buffer 2 (2x lysis buffer) contained 1% Triton X-100 in PBS (without CaCl₂ and MgCl₂).

Buffer 3 (assay buffer) contained 50 mM potassium phosphate buffer (pH 7.0); 800 mM potassium fluoride; 0.2% BSA (always added fresh).

### Procedure:

On day 1, the cells were seeded in 96-well plates (3x10⁴ cells per well hFSHR clone 16 cells (CHO cells stably transfected with the human FSH receptor in 150 µl of medium). The next day, test substance dilutions were made up. For this purpose, all the substances were diluted in ice-cold buffer 1 (with or without hFSH), and the substance dilutions were placed on ice until applied to the cells.

The cell supernatant was then aspirated off, and the cells were washed 2x with 200 µl of buffer 1. The cells were treated with 60 µl of the appropriate substance concentrations at 37°C for 2h. The cells were then lysed with 60 µl of buffer 2 (put onto the supernatant) (on a plate shaker at RT for 30 min).

The test conjugates (XL-665 and anti-cAMP cryptate) were diluted in buffer 3 in accordance with the manufacturers' information. The actual mixture for measurement was pipetted into a black 96-well plate (in each case 15 µl of the cell lysate diluted with 35 µl of buffer 1; firstly 25 µl of XL-665 conjugate were pipetted and, after 10 min, 25 µl of the anti-cAMP cryptate were added). This is followed by incubation at RT for 90 minutes. The measurement was carried out in a PheraStar (BMG).

### Tissue culture conditions

1) hFSHr clone 16 Ham's F12
   PSG
   10% FCS
   700 µg/ml G 418 (Geneticin) from PAA.

Dose-effect curve (hFSH) for the human receptor: 1 e-8, 3e-9, 1 e-9, 3e-10, 1 e-1 0, 3e-11, 1 e-11, 3e-12 mol/l.

The test substances were employed in suitable dilutions in the absence (test for agonism) and in the presence of 1 e-9 mol/l hFSH.

### Evaluation

The values of the well ratio were averaged and then entered directly in SigmaPlot versus the concentrations. The maximum and minimum values were determined for each plate, and half the difference is to be regarded as IC₅₀.

The test results (Table 1) show that the compounds according to the invention have an FSH-antagonistic effect.

**Table 1. FSH-antagonistic effect of selected compounds in the HTRF assay**

| Compound [Ex. #] | IC₅₀ |
|---|---|
| 4 | 2 µM |
| 13 | 8 µM |
| 18 | 200 nM |
| 19 | 4 µM |
| 25 | 900 nM |
| 30 | 200 nM |
| 32 | 3 µM |
| 33 | 70 nM |
| 34 | 1.5 µM |
| 38 | 600 nM |
| 39 | 6 µM |
| 40 | 750 nM |
| 42 | 350 nM |
| 46 | 2.5 µM |

Being antagonists of the FSH receptor, compounds of the general formula I or pharmaceutically acceptable salts thereof can thus be used for the manufacture of medicaments to be used for the fertility control in male and/or in a female animals, in particular in men and/or women; as well as for the treatment and/or prevention of osteoporosis.

### Dosage

Satisfactory results are generally to be expected if the daily doses comprise a range from 5 µg to 50 mg of the compound according to the invention per kg of body weight. A recommended daily dose for larger mammals, for example humans, is in the range from 10 µg to 30 mg per kg of body weight. Suitable dosages for the compounds according to the invention are from 0.005 to 50 mg per day per kg of body weight, depending on the age and constitution of the patient, it being possible to administer the necessary daily dose by single or multiple delivery.

Pharmaceutical products based on the novel compounds are formulated in a manner known per se by processing the active ingredient with the carrier substances, fillers, substances which influence disintegration, binders, humectants, lubricants, absorbents, diluents, test modifiers, colorants etc. which are used in pharmaceutical technology, and converting into the desired administration form. Reference should be made in this connection to Remington's Pharmaceutical Science, 15th ed. Mack Publishing Company, East Pennsylvania (1980).

Suitable for oral administration are in particular tablets, coated tablets, capsules, pills, powders, granules, pastilles, suspensions, emulsions or solutions. Preparations for injection and infusion are possible for parenteral administration. Appropriately prepared crystal suspensions can be used for intraarticular injection. Aqueous and oily solutions for injection or suspensions and corresponding depot preparations can be used for intramuscular injection. The novel compounds can be used for rectal administration in the form of suppositories, capsules, solutions (e.g. in the form of enemas) and ointments both for systemic and for local therapy. Formulations possible for topical application are gels, ointments, greasy ointments, creams, pastes, dusting powders, milk and tinctures. The dosage of the compounds of the general formula I in these preparations should be 0.01% - 20% in order to achieve an adequate pharmacological effect. Topical use can also take place by means of a transdermal system, for example a patch.

The invention likewise encompasses the compounds according to the invention of the general formula I as therapeutic active ingredient. The invention further includes the compounds according to the invention of the general formula I as therapeutic active ingredients together with pharmaceutically suitable and acceptable excipients and carriers. The invention likewise encompasses a pharmaceutical composition which comprises one of the pharmaceutically active compounds according to the invention or mixture thereof and a pharmaceutically suitable salt or pharmaceutically suitable excipients and carriers.

The present invention therefore also relates to pharmaceutical compositions which comprise at least one compound of the general formula I, where appropriate together with pharmaceutically suitable excipients and/or carriers.

Suitable for forming pharmaceutically suitable salts of the compounds according to the invention of the general formula I are, by methods known to the skilled person, as inorganic acids inter alia hydrochloric acid, hydrobromic acid, sulphuric acid and phosphoric acid, nitric acid, as carboxylic acids inter alia acetic acid, propionic acid, hexanoic acid, octanoic acid, decanoic acid, oleic acid, stearic acid, maleic acid, fumaric acid, succinic acid, benzoic acid, ascorbic acid, oxalic acid, salicylic acid, tartaric acid, citric acid, lactic acid, glycolic acid, malic acid, mandelic acid, cinnamic acid, glutamic acid, aspartic acid, and as sulphonic acids inter alia methanesulphonic acid, ethanesulphonic acid, toluenesulphonic acid, benzenesulphonic acid and naphthalenesulphonic acid.

These pharmaceutical compositions and medicaments may be intended for oral, rectal, subcutaneous, transdermal, percutaneous, intravenous or intramuscular administration. They comprise besides conventional carriers and/or diluents at least one compound of the general formula I.

The medicaments of the invention are produced using the customary solid or liquid carriers or diluents and the excipients customarily used in pharmaceutical technology, in accordance with the desired mode of administration with a suitable dosage in a known manner. The preferred preparations consist of a dosage form which is suitable for oral administration. Examples of such dosage forms are tablets, film-coated tablets, sugar-coated tablets, capsules, pills, powders, solutions or suspensions or else depot forms.

The pharmaceutical compositions which comprise at least one of the compounds according to the invention are preferably administered orally.

Parenteral preparations such as solutions for injection are also suitable. Preparations which may also be mentioned for example are suppositories.

Appropriate tablets can be obtained for example by mixing the active ingredient with known excipients, for example inert diluents such as dextrose, sugar, sorbitol, mannitol, polyvinylpyrrolidone, disintegrants such as maize starch or alginic acid, binders such as starch or gelatine, lubricants such as magnesium stearate or talc and/ or agents to achieve a depot effect such as carboxylpolymethylene, carboxylmethylcellulose, cellulose acetate phthalate or polyvinyl acetate. The tablets may also consist of a plurality of layers.

Correspondingly, coated tablets can be produced by coating cores which have been produced in analogy to the tablets with agents normally used in tablet coatings, for example polyvinylpyrrolidone or shellac, gum Arabic, talc, titanium oxide or sugar. The tablet coating may also consist of a plurality of layers, it being possible to use the excipients mentioned above for tablets.

Solutions or suspensions with the compounds according to the invention of the general formula I may additionally comprise taste-improving agents such as saccharin, cyclamate or sugar and, for example, flavourings such as vanillin or orange extract. They may additionally comprise suspending aids such as sodium carboxymethylcellulose or preservatives such as p-hydroxybenzoates.

Capsules comprising the compounds of the general formula I can be produced for example by the compound(s) of the general formula I being mixed with an inert carrier such as lactose or sorbitol and encapsulated in gelatine capsules.

Suitable suppositories can be produced for example by mixing with carriers intended for this purpose, such as neutral fats or polyethylene glycol or derivatives thereof.

The present invention also relates to processes for preparing the compounds according to the invention.

Compounds of the general formula I can be prepared as shown in Scheme 1 by an amide-formation reaction between the tetrahydrocarbazol derivative VI and the carboxylic acid VII. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N-*ethylcarbodiimide hydrochloride) / HOBt (1-hydroxy-1*H*-benzotriazole). It is possible as alternative for the carboxylic acid VII to be converted, where appropriate in the presence of a base, into the carbonyl chloride and reacted with the tetrahydrocarbazol VI to give the product of the general formula I.

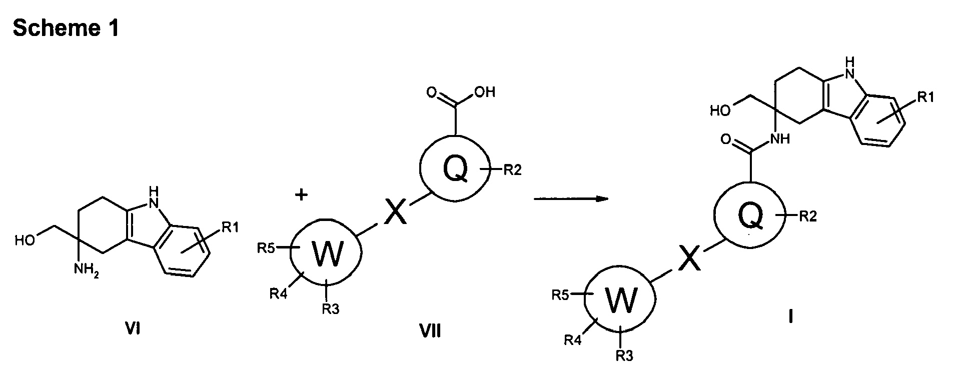

Compounds of general formula II can be prepared as shown in Scheme 2 by an amide-formation reaction between the tetrahydrocarbazol derivative VI and carboxylic acid VIII. Reagents suitable for this purpose are all known peptide-coupling reagents which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N'*-ethylcarbodiimide hydrochloride) / HOBt (1-hydroxy-1*H*-benzotriazole). It is possible as alternative for the carboxylic acid VIII to be converted, where appropriate in the presence of a base, into the carbonyl chloride and reacted with the tetrahydrocarbazol VI to give the product of the general formula II. 1.

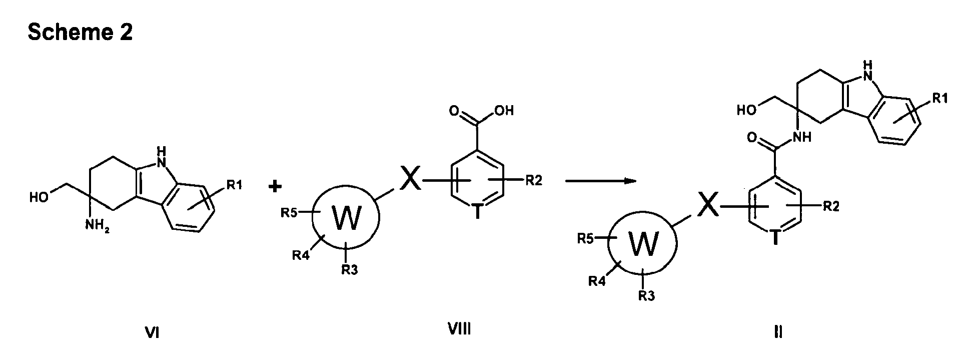

Compounds of general formula III can be prepared as shown in Scheme 3 by an amide-formation reaction between the tetrahydrocarbazol derivative VI and carboxylic acid IX. Reagents suitable for this purpose are all suitable peptide-coupling reagents which are known to the skilled person and which convert the carboxylic acid, where appropriate in the presence of a base, into an intermediate active ester, for example PyBOP ([(1*H-*benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N'*-ethylcarbodiimide hydrochloride) / HOBt (1-hydroxy-1*H-*benzotriazole). It is possible as alternative for the carboxylic acid IX to be converted, where appropriate in the presence of a base, into the carbonyl chloride and reacted with the tetrahydrocarbazol VI to give the product of the general formula III.

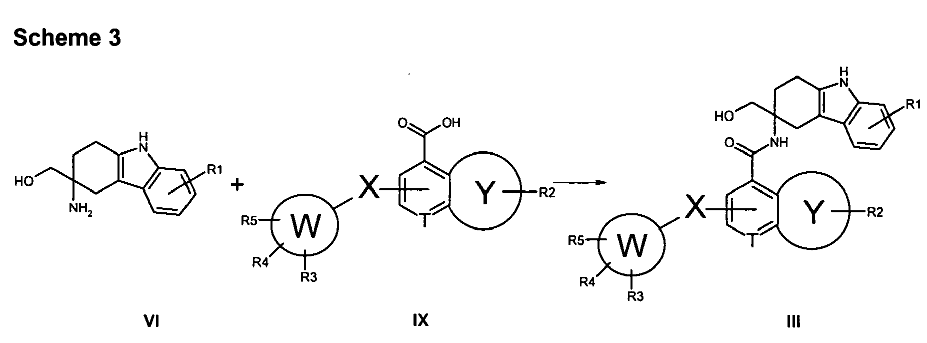

Compounds of general formula IV can be prepared as shown in Scheme 4 by an amide-formation reaction between the tetrahydrocarbazol derivative VI and carboxylic acid X. Reagents suitable for this purpose are all suitable peptide-coupling reagents which are known to the skilled person and which convert the carboxylic acid, where appropriate in the presence of a base, into an intermediate active ester, for example PyBOP ([(1*H-*benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N*'-ethylcarbodiimide hydrochloride) / HOBt (1-hydroxy-1*H-*benzotriazole). It is possible as alternative for the carboxylic acid X to be converted, where appropriate in the presence of a base, into the carbonyl chloride and reacted with the tetrahydrocarbazol VI to give the product of the general formula IV.

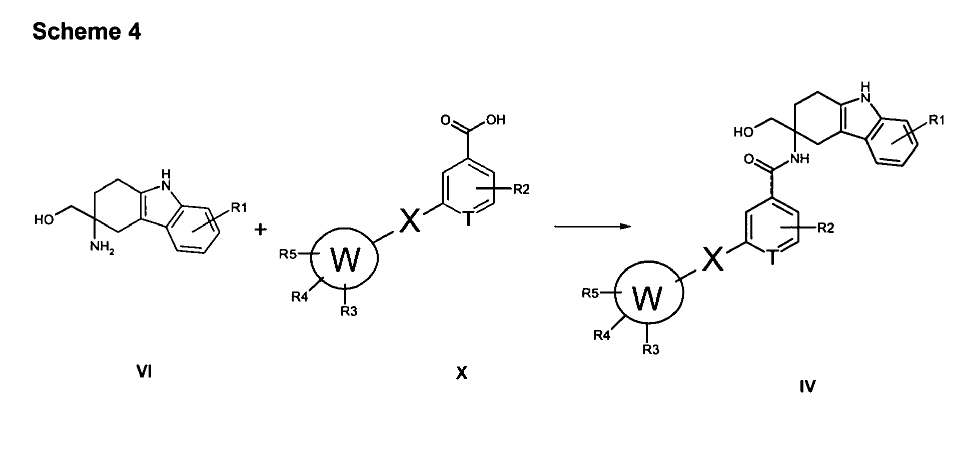

Compounds of general formula V can be prepared as shown in Scheme 5 by an amide-formation reaction between the tetrahydrocarbazol derivative VI and carboxylic acid XI. Reagents suitable for this purpose are all suitable peptide-coupling reagents which are known to the skilled person and which convert the carboxylic acid, where appropriate in the presence of a base, into an intermediate active ester, for example PyBOP ([(1*H-*benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyluronium hexafluorophosphate), HBTU (2-(1*H*-benzotriazol-1-yl)-1,1,3,3-tetramethyluronium hexafluorophosphate), EDC (*N*-[3-(dimethylamino)propyl]-*N*'-ethylcarbodiimide hydrochloride) / HOBt (1-hydroxy-1*H-*benzotriazole). It is possible as alternative for the carboxylic acid XI to be converted, where appropriate in the presence of a base, into the carbonyl chloride and reacted with the tetrahydrocarbazol VI to give the product of the general formula V.

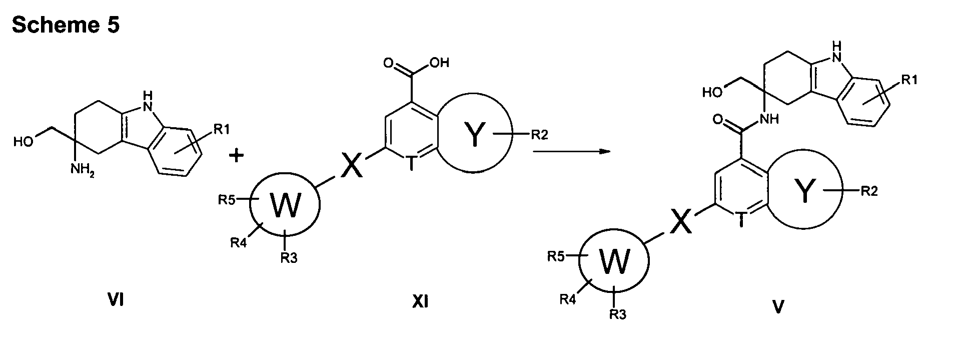

Likewise compounds of general formula I can be prepared as shown in Scheme 6 by a tandem acetal deprotection - Fischer indole synthesis reaction between the acetal derivative XIII and the corresponding phenyl hydrazine XII. Reagents suitable for this purpose are all suitable inorganic / organic protic or Lewis acids which are known to the skilled person to activate the acetal. Examples include ZnCl₂, ZnBr₂, BF₃ ether complex, para-toluene sulfonic acid, trifluoroacetic acid, HCl aq. It is also possible as an alternative to perform the reaction in two steps, i.e. cleave the acetal and isolate the intermediate ketone, which is in turn converted in a separate reaction vessel into compounds of formula I, where appropriate in the presence of a suitable inorganic/organic protic or Lewis acid.

The groups R' and R" in formula XIII are typically C₁-C₆-alkyl groups; or R' and R" together form a cyclic acetal such as for isopropylidene, cyclohexylidene, cyclopentylidene. Alternatively, R' and R" are both linked/bonded to a solid phase resin, for example glycerol resin.

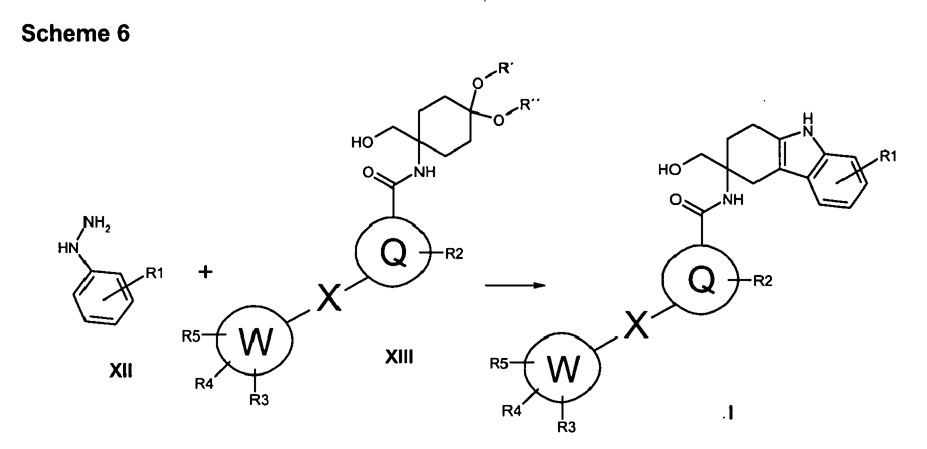

Likewise compounds of general formula II can be prepared as shown in Scheme 7 by a tandem acetal deprotection - Fischer indole synthesis reaction between the acetal derivative XIV and the corresponding phenyl hydrazine XII. Reagents suitable for this purpose are all suitable inorganic / organic protic or Lewis acids which are known to the skilled person to activate the acetal. Examples include ZnCl₂, ZnBr₂, BF₃ ether complex, para-toluene sulfonic acid, trifluoroacetic acid, HCl aq. It is also possible as an alternative to perform the reaction in two steps, i.e. cleave the acetal and isolate the intermediate ketone, which is in turn converted in a separate reaction vessel into compounds of formula II, where appropriate in the presence of a suitable inorganic/organic protic or Lewis acid.

The groups R' and R" in formula XIV are typically C₁-C₆-alkyl groups; or R' and R" together form a cyclic acetal such as for isopropylidene, cyclohexylidene, cyclopentylidene. Alternatively, R' and R" are both linked/bonded to a solid phase resin, for example glycerol resin.

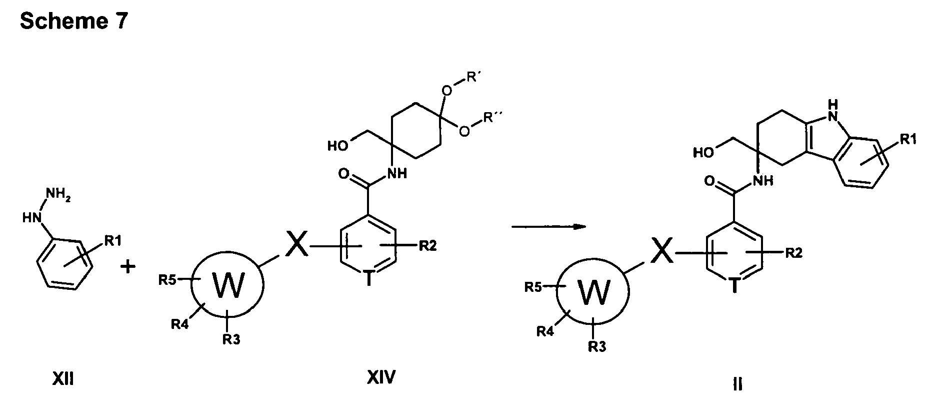

Likewise compounds of general formula III can be prepared as shown in Scheme 8 by a tandem acetal deprotection - Fischer indole synthesis reaction between the acetal derivative XV and the correspondinding phenyl hydrazine XII. Reagents suitable for this purpose are all suitable inorganic / organic protic or Lewis acids which are known to the skilled person to activate the acetal. Examples include ZnCl₂, ZnBr₂, BF₃ ether complex, para-toluene sulfonic acid, trifluoroacetic acid, HCl aq. It is also possible as an alternative to perform the reaction in two steps, i.e. cleave the acetal and isolate the intermediate ketone, which is in turn converted in a separate reaction vessel into compounds of formula III, where appropriate in the presence of a suitable inorganic/organic protic or Lewis acid.

The groups R' and R" in formula XV are typically C₁-C₆-alkyl groups; or R' and R" together form a cyclic acetal such as for isopropylidene, cyclohexylidene, cyclopentylidene. Alternatively, R' and R" are both linked/bonded to a solid phase resin, for example glycerol resin.

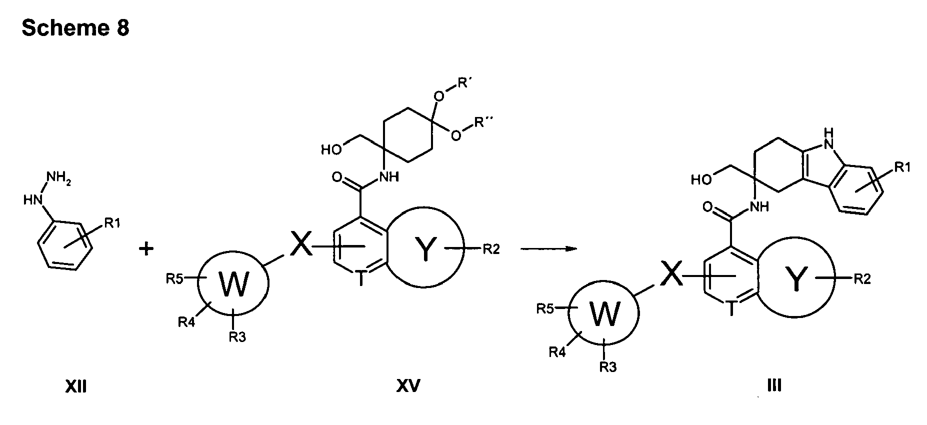

Likewise compounds of general formula IV can be prepared as shown in Scheme 9 by a tandem acetal deprotection - Fischer indole synthesis reaction between the acetal derivative XVI and the corresponding phenyl hydrazine XII. Reagents suitable for this purpose are all suitable inorganic / organic protic or Lewis acids which are known to the skilled person to activate the acetal. Examples include ZnCl₂, ZnBr₂, BF₃ ether complex, para-toluene sulfonic acid, trifluoroacetic acid, HCl aq. It is also possible as an alternative to perform the reaction in two steps, i.e. cleave the acetal and isolate the intermediate ketone, which is in turn converted in a separate reaction vessel into compounds of formula IV, where appropriate in the presence of a suitable inorganic/organic protic or Lewis acid.

The groups R' and R" in formula XVI are typically C₁-C₆-alkyl groups; or R' and R" together form a cyclic acetal such as for isopropylidene, cyclohexylidene, cyclopentylidene. Alternatively, R' and R" are both linked/bonded to a solid phase resin, for example glycerol resin.

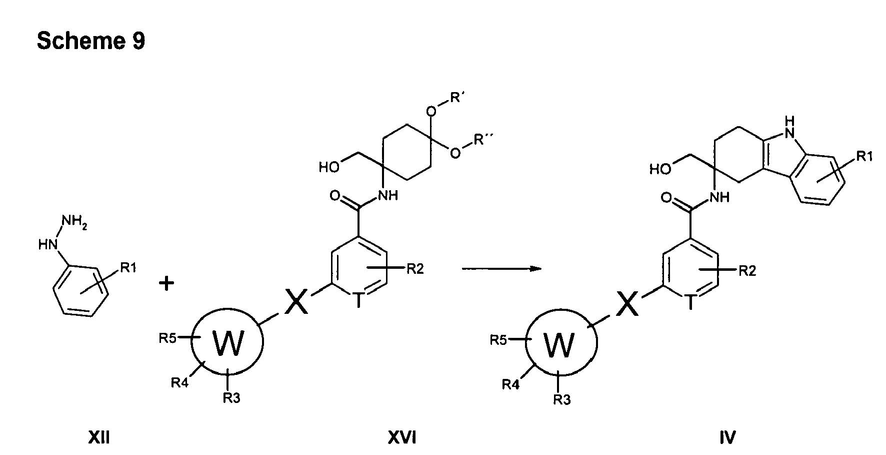

Likewise compounds of general formula V can be prepared as shown in Scheme 6 by a tandem acetal deprotection - Fischer indole synthesis reaction between the acetal derivative XVII and the corresponding phenyl hydrazine XII. Reagents suitable for this purpose are all suitable inorganic / organic protic or Lewis acids which are known to the skilled person to activate the acetal. Examples include ZnCl₂, ZnBr₂, BF₃ ether complex, para-toluene sulfonic acid, trifluoroacetic acid, HCl aq. It is also possible as an alternative to perform the reaction in two steps, i.e. cleave the acetal and isolate the intermediate ketone, which is in turn converted in a separate reaction vessel into compounds of formula V, where appropriate in the presence of a suitable inorganic/organic protic or Lewis acid.

The groups R' and R" in formula XVII are typically C₁-C₆-alkyl groups; or R' and R" together form a cyclic acetal such as for isopropylidene, cyclohexylidene, cyclopentylidene. Alternatively, R' and R" are both linked/bonded to a solid phase resin, for example glycerol resin.

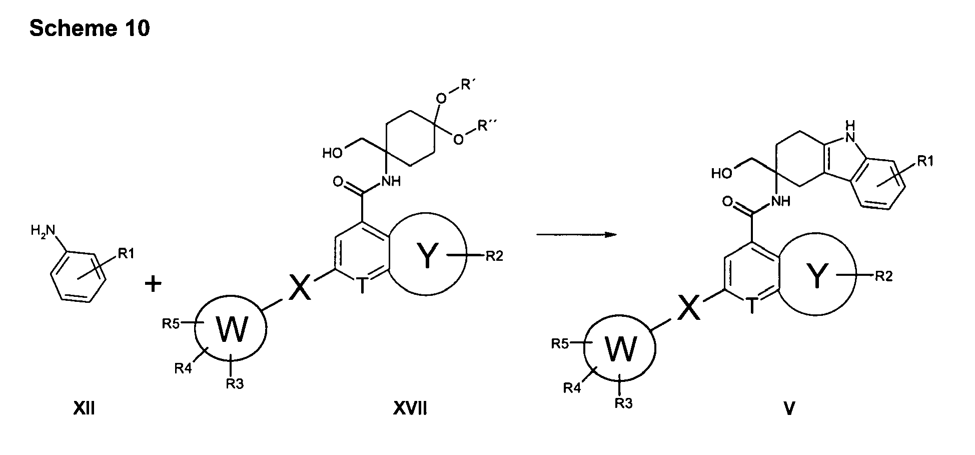

The compounds according to the invention of the general formula I can be prepared as described below.

### Abbreviations used:

- ACN: Acetonitrile
- DIBAC: Diisobutylaluminium hydride
- DMF: N,N-Dimethylformamide
- EDC: N-Ethyl-N'-(3-dimethylaminopropyl)carbodiimide
- EtOH: Ethanol
- HATU: O-(7-Azabenzotriazol-1-yl)-N,N,N',N'-tetramethyluronium hexafluorophosphate
- FMOC: (9H-Fluoren-9-ylmethoxy)carbonyl
- HOBt: 1-Hydroxy-1H-benzotriazole
- MeCN: Acetonitrile
- MeOH: Methanol
- MTBE: Methyl tert-butyl ether
- NMM: 4-methylmorpholine
- NMP: N-Methylpyrrolidinone
- pTsOH: para-toluene sulfonic acid
- Rf: Reflux
- RT: Room temperature
- TBAF: Tetrabutylammonium fluoride
- TFA: Trifluoroacetic acid
- THF: Tetrahydrofuran

Compounds of the general formula I can in principle be prepared as shown in Scheme 11 by an amide-formation reaction between a tetrahydrocarbazol derivative VI and a carboxylic acid VII. The reagents typically used for the coupling are EDC and HOBt.

The tetrahydrocarbazoles VI can be prepared analogously to those described in WO2005/33099, example 5 A and B.

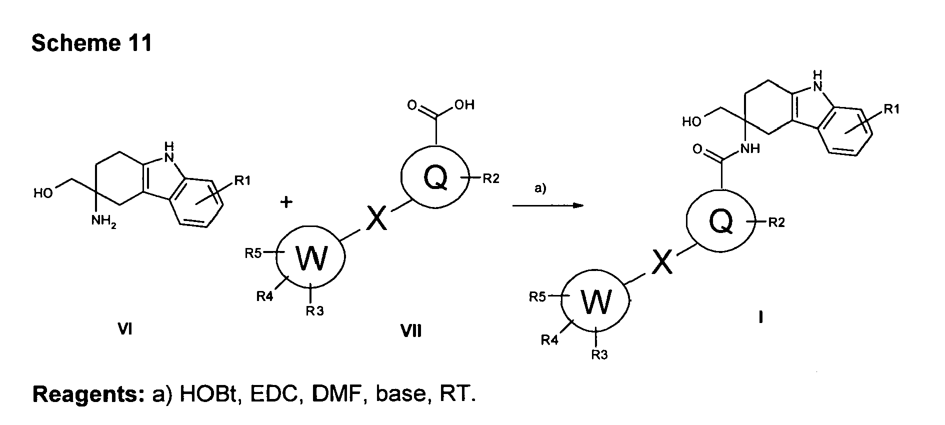

Compounds of the general formula I can in principle be prepared as shown in Scheme 12. Ketone XVIII is linked to glycerol resin XIX and forms compound XX. The carboxylic acid XX is then reduced to the corresponding alcohol XXI by sodium borohydride. After deprotection of the FMOC group with piperidine the free amine XXII is reacted with an appropriate carboxylic acid of formula VII to form amide XXIII. In a one pot reaction the acetal XXIII is cleaved from the resin and converted via a Fischer indole cyclyzation with the corresponding phenyl hydrazines XII to compounds of the general formula I.

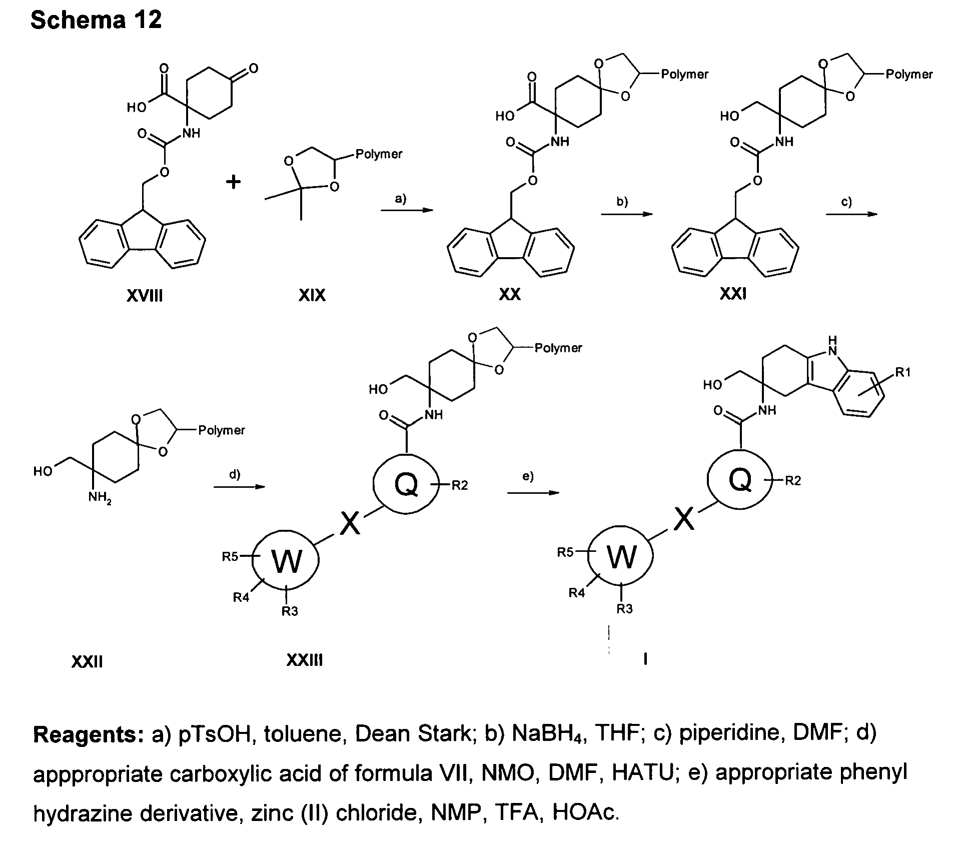

The carboxylic acids of the general formula VII can be prepared as shown in Scheme 13 by a Suzuki reaction between a boronic acid XXIV or XXVII and corresponding halogen compound XXV or XXVI (Hal = I, Br, Cl) to form ester XXVIII which can be converted to carboxylic acid VII via an ester hydrolysis reaction with KOH in methanol.

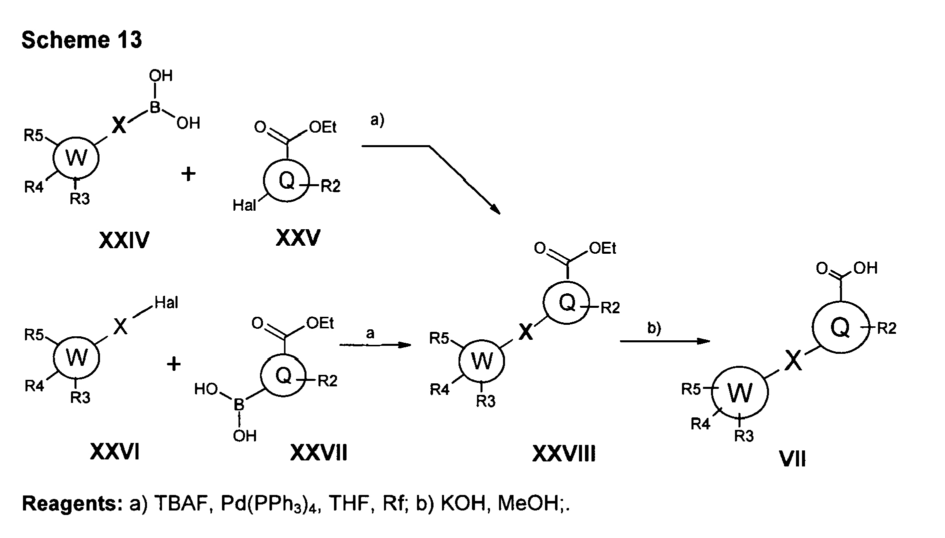

The carboxylic acid derivatives of formula XXXII can in principle be prepared according to Scheme 14 via a Sonogashira type coupling of acetylenes XXIX or XXXIV with their corresponding aryl halides XXX or XXXIII with subsequent hydrolysis of the resulting carboxylic esters XXXI.

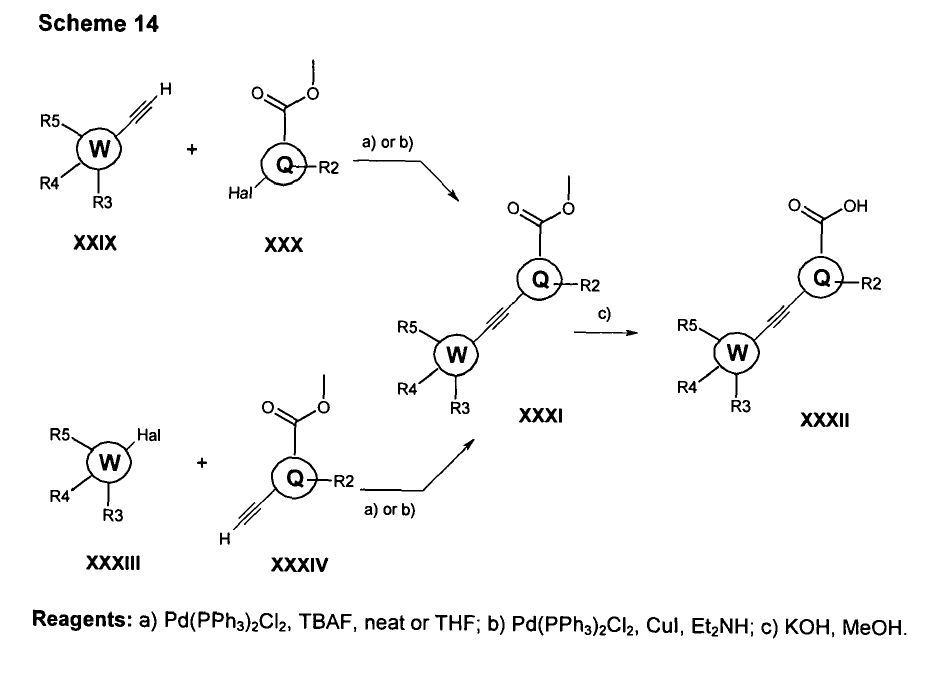

Carboxylic acids of the formula XXXVII can be prepared as shown in Scheme 15 in a so-called Pfitzinger reaction from a methyl ketone XXXV and an isatin derivative XXXVI.

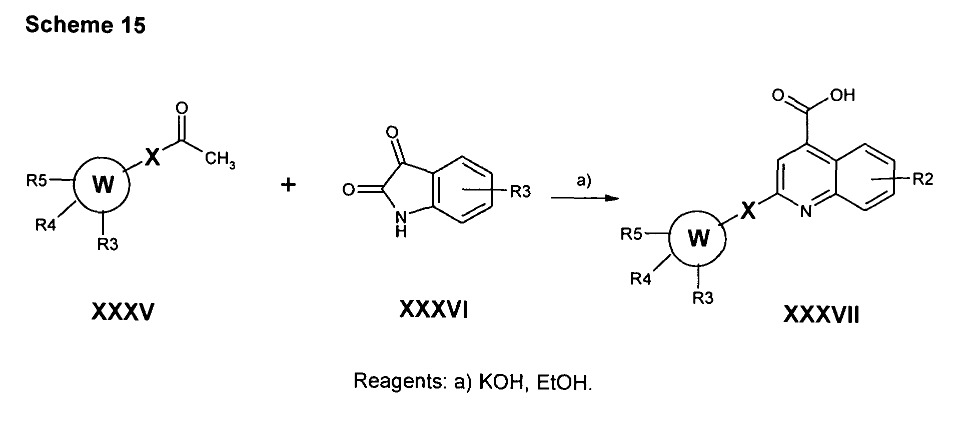

### Synthesis of the compounds according to the invention

### Preparation of the carboxylic acids

6-Methoxy-2-(3,4,5-trimethoxy-phenyl)-quinoline-4-carboxylic acid and 6-Methoxy-2-phenyl-quinoline-4-carboxylic acid were prepared in analogy to WO 2007/017289, example 13a.

4-Isopropoxy-3',4'-dimethoxy-biphenyl-3-carboxylic acid, 3'-Chloro-4-isopropoxy-4'-methylcarbamoyl-biphenyl-3-carboxylic acid and 5-Benzofuran-2-yl-2-isopropoxybenzoic acid were prepared in analogy to WO 2007/017289, example 39a and 39b.

### Preparation of 2-Isopropoxy-5-(2-methoxy-phenylethynyl)-benzoic acid

### a) 5-lodo-2-isopropoxy-benzoic acid methyl ester

A suspension of 5-lodo-2-hydroxy benzoic acid methyl ester (50 g), potassium carbonate (74,6 g) and iso-propyl iodide (89,9 ml) in acetone (500 ml) was stirred under reflux overnight. The reaction mixture was allowed to cool down to ambient temperature and the solid was removed by filtration. The filtrate was evaporated and the title compound was obtained in 96 % yield (55,1 g). **¹H-NMR (CDCl₃):** 8.02 d (J = 2.3 Hz, 1H); 7.67 dd (J = 2.5 Hz / 8.9 Hz, 1 H); 6.74 d (J = 8.9 Hz, 1 H); 4.54 m (1 H); 3.87 s (3H); 1.36 m (6H).

### b) 2-Isopropoxy-5-(2-methoxy-phenylethynyl)-benzoic acid methyl ester

5-lodo-2-isopropoxy-benzoic acid methyl ester (500 mg), 2-methoxy-phenyl acetylene (0.40 ml), palladium dichlorobis(triphenylphosphine) (12 mg) and Cul (6 mg) in diethylamine (10 ml) were stirred at 60°C for 12 hours. The reaction mixture was concentrated and extracted with ethylacetate / water. The combined organic layers were dried over sodium sulphate and the solvent was evaporated. The title compound was obtained in 64 % yield after flash chromatography. **¹H-NMR (CDCl₃):** 7.97 d (J = 2.3 Hz, 1 H); 7.59 dd (J = 2.3 Hz /8.7 Hz, 1 H); 7.47 dd (J = 7.5 Hz /1.7 Hz, 1 H); 7.30 m (1 H); 6.96 m (3H); 4.62 m (1 H); 3.91 s (3H); 3.88 s (3H); 1.38 d (*J* =8.7 Hz, 6H).

### c) 2-Isopropoxy-5-(2-methoxy-phenylethynyl)-benzoic acid

A solution of 2-Isopropoxy-5-(2-methoxy-phenylethynyl)-benzoic acid methyl ester (280 mg) in methanolic KOH solution (10%) was stirred at ambient temperature overnight.The solvent was distilled off and the remaining solid was dissolved in water and extracted with ethylacetate. The water phase was acidified by addition of HCl (2 molar) and the water phase was extracted with ethylacetate (3x 50 ml). The combined organic layers were dried over sodium sulphate and evaporated to dryness. The title compound was obtained in 90 % yield. **¹H-NMR (DMSO-d₆):** 8.45 d (J = 2.3 Hz, 1 H); 7.75 dd (J = 2.3 Hz / 8.7 Hz, 1 H); 7.52 dd (J = 1.7 Hz / 7.5 Hz, 1 H); 7.37 m (1 H); 7.08 d (J = 8.7 Hz, 1 H); 6.97 m (2H); 4.94 m (1 H); 3.96 s (3H); 1.55 d (J = 6.0 Hz, 6H).

### General procedure:

### Loading of 1-[[(9H-Fluoren-9-ylmethoxy)carbonyl]aminol-4-oxocyclohexane carboxylic acid to (+)-1-(2,3-isoprropylidene) glycerol resin

17 g of (+)-1-(2,3-isoprropylidene) glycerol resin (loading ~ 1-1.8 mmol/g) were refluxed for 24 h with 34 g (3 eq) 1-[[(9H-Fluoren-9-ylmethoxy)carbonyl]amino]-4-oxocyclohexane carboxylic acid and 34 mg p-toluenesulfonic acid (0.006 eq) in toluene with azetropic water removal by a dean-stark trap. The resin was filtered, washed with pyridine, pyridine/DMF 1:1, H₂O, EtOH and Ether and dried. Resin loading was determined by cleavage of 100 mg resin with 95% TFA/H₂O and gravimetric yield determination. Loading 0.89 mmol/g.

### Carboxylic acid reduction to alcohol

23.5 g (21 mmol) loaded resin was swollen in 200 mL THF and 1 eq of N-Methylmorpholine (2.3 mL) and 1 eq of Isobutylchloroformiate (2.7 mL) were added. After shaking for 6 h, a preformed suspension of 3.96 g NaBH4 (5 eq) in 100 mL THF was added and the resin was shaken overnight. 250 mL MeOH were added, and the resin was shaken for 1 h. The resin was filtered, and washed with THF, THF/sat. Na₂SO₄ solution (1:1), THF/H₂O (1:1), THF, MeOH and Ether. For complete reduction, the whole procedure was repeated once

### Deprotection of FMOC group and acid coupling

5.62 g alcohol resin (5 mmol) were treated with 50 mL 20% Piperidine in DMF and shaken for 1 h at room temperature. After filtration, the procedure was repeated once. The resin was filtered, washed with 50 mL of DMF, H₂O, DMF, Isopropanol, Ether and dried. The Fmoc-deprotected resin was swollen in 25 mL DMF, and treated with 2 eq Carboxylic acid, 4 eq 3 M N-Methylmorpholine in DMF (7.44 mL), 2 eq HATU (0.5 M in DMF, 22.4 mL) and was shaken for 2 h at room temperature. The resin was filtered and washed with 50 mL of DMF, DCM, MeOH, ether and dried.

### Tetrahydrocarbazole formation and cleavage

0.2 mmol (~0.16 g) of acylated alcohol resin were treated with 5eq phenyl hydrazine derivative and 5 eq ZnCl₂ (2 mL, dissolved 0.5 M in NMP/TFA/HOAc 3:2:5) and shaken for 20 h at 70°C. The resin was filtered, washed once with 1 mL NMP and the combined filtrate is neutralized with 1 N NaOH. After evaporation, the products were purified by preparative HPLC.

Machine: Analytical 4 channel MUX system with CTC Pal injector, Waters 1525 pumps, Waters 2488 UV detector and Waters ZQ 2000 single quad MS detector.

The following compounds were obtained in analogy to the preparation methods described above:

| **Ex.** | **Product; reagents** | **HPLC-MS** | **Structure** |
|---|---|---|---|
| **1** | 6-Methoxy-2-(3,4,5-trimethoxy-phenyl)-quinoline-4-carboxylic acid ((S)-3-hydroxy-methyl-2,3,4,9-tetrahydro-1H-carbazol-3-yl)-amide; *6-Methoxy-2-(3,4,5-trimethoxy-phenyl)-quinoline-4-carboxylic acid and Phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 569 Retention time: 4.05 min. | 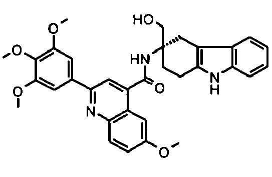 |
| **2** | 6-Methoxy-2-phenyl-quinoline-4-carboxylic acid (5,8-dichloro-3-hydroxymethyl-2,3,4,9-tetrahydro-1 H-carbazol-3-yl)-amide; *6-Methoxy-2-phenyl-quinoline-4-carboxylic acid and 2,5-Dichloro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). 3Molecular peak (ESI, M+1): 547 Retention time: 4.13 min. | 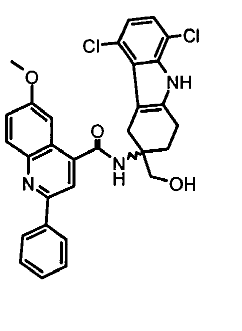 |
| **3** | 6-Methoxy-2-phenyl-quinoline-4-carboxylic acid (3-hydroxymethyl-8-trifluoromethyl-2,3,4,9-tetrahydro-1H-carbazol-3-yl)-amide; *6-Methoxy-2-phenyl-quinoline-4-carboxylic acid and 2- Trifluoromethyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 547 Retention time: 4.08 min. | 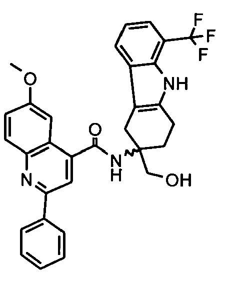 |
| **4** | 6-Methoxy-2-phenyl-quinoline-4-carboxylic acid (3-hydroxymethyl-2,3,4,9-tetrahydro-1 H-carbazol-3-yl)-amide; *6-Methoxy-2-phenyl-quinoline-4-carboxylic acid and Phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 479 Retention time: 3.68 min. | 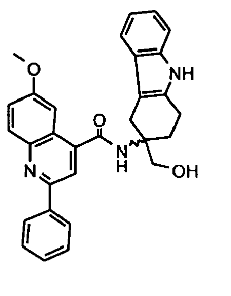 |
| **5** | 6-Hydroxymethyl-6-[(6-methoxy-2-phenyl-quinoline-4-carbonyl)-amino]-6,7,8,9-tetrahydro-5H-carbazole-3-carboxylic acid; *6-Methoxy-2-phenyl-quinoline-4-carboxylic acid and 4-Carboxy-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 523 Retention time: 4.44 min. | 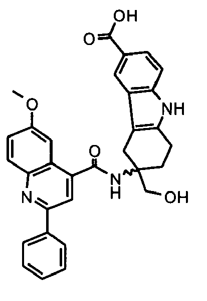 |
| **6** | 6-Methoxy-2-phenyl- quinoline-4-carboxylic acid (8-chloro-3-hydroxymethyl-2,3,4,9-tetrahydro-1H-carbazol-3-yl)-amide; *6-Methoxy-2-phenyl-quinoline-4-carboxylic acid and 2-Chloro-phenyl-hydrazine* | I HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 513 Retention time: 3.95 min. | 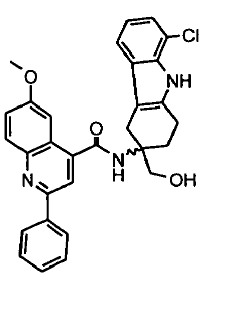 |
| **7** | 6-Methoxy-2-phenyl-quinoline-4-carboxylic acid (5-chloro-3-hydroxymethyl-6-methyl-2,3,4,9-tetrahydro-1H-carbazol-3-yl)-amide; *6-Methoxy-2-phenyl-quinoline-4-carboxylic acid and 3-Chloro-4-methyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 527 Retention time: 4.10 min. | 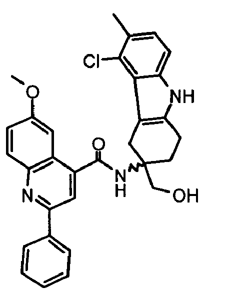 |
| **8** | 6-Methoxy-2-phenyl-quinoline-4-carboxylic acid (6-chloro-3-hydroxymethyl-8-methyl-2,3,4,9-tetrahydro-1H-carbazol-3-yl)-amide; *6-Methoxy-2-phenyl-quinoline-4-carboxylic acid and 4-Chloro-2-methyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 527 Retention time: 5.76 min. | 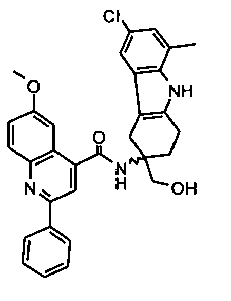 |
| **9** | 6-Methoxy-2-phenyl-quinoline-4-carboxylic acid (3-hydroxymethyl-5,8-dimethyl-2,3,4,9-tetrahydro-1H-carbazol-3-yl)-amide; *6-Methoxy-2-phenyl-quinoline-4-carboxylic acid and 2,5-Dimethyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 507 Retention time: 3.98 min. | 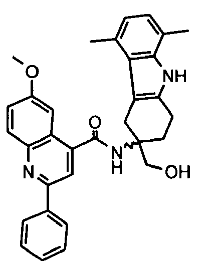 |
| **10** | 6-Methoxy-2-phenyl-quinoline-4-carboxylic acid (3-hydroxymethyl-6-trifluoromethyl-2,3,4,9-tetrahydro-1H-carbazol-3-yl)-amide; *6-Mefhoxy-2-phenyl-quinoline-4-carboxylic acid and 4-Trifluoromethyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 547 Retention time: 4.02 min. | 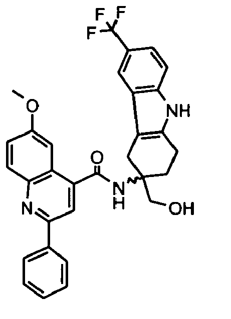 |
| **11** | 6-Methoxy-2-phenyl-quinoline-4-carboxylic acid (3-hydroxymethyl-6-trifluoromethoxy-2,3,4,9-tetrahydro-1H-carbazol-3-yl)-amide; *6-Methoxy-2-phenyl-quinoline-4-carboxylic acid and 4-Trifluoromethyloxy-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 563 Retention time: 5.74 min. | 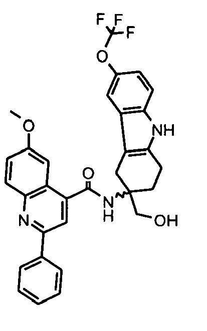 |
| **12** | 6-Methoxy-2-phenyl-quinoline-4-carboxylic acid (8-fluoro-3-hydroxymethyl-2,3,4,9-tetrahydro-1H-carbazol-3-yl)-amide; *6-Methoxy-2-phenyl-quinoline-4-carboxylic acid and 2-Fluor-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 497 Retention time: 3.75 min. | 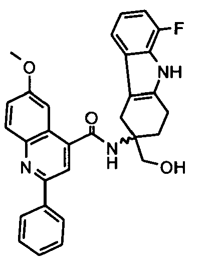 |
| **13** | 6-Methoxy-2-phenyl-quinoline-4-carboxylic acid (7-chloro-6-fluoro-3-hydroxymethyl-2,3,4,9-tetrahydro-1 H-carbazol-3-yl)-amide; *6-Methoxy-2-phenyl-quinoline-4-carboxylic acid 3-Chloro-4-fluoro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 531 Retention time: 3.95 min. | 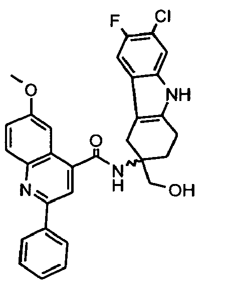 |
| **14** | 6-Methoxy-2-phenyl-quinoline-4-carboxylic acid (3-hydroxymethyl-6-sulfamoyl-2,3,4,9-tetrahydro-1H-carbazol-3-yl)-amide; *6-Methoxy-2-phenyl-quinoline-4-carboxylic acid and 4-Hydrazino-benzenesulfonamide* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 558 Retention time: 3.02 min. | 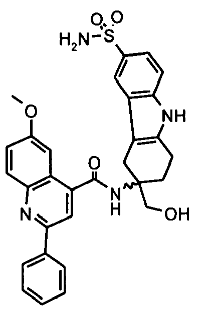 |
| **15** | 6-Methoxy-2-phenyl-quinoline-4-carboxylic acid (5,6-dichloro-3-hydroxymethyl-2,3,4,9-tetrahydro-1H-carbazol-3-yl)-amide; *6-Methoxy-2-phenyl-quinoline-4-carboxylic acid and 3,4-Dichloro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 547 Retention time: 5.74 min. | 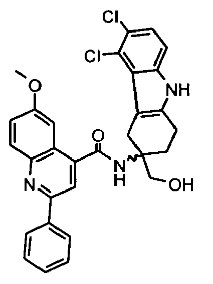 |
| **16** | 4-Isopropoxy-3',4'-dimethoxy-biphenyl-3-carboxylic acid (3-hydroxymethyl-2,3,4,9-tetrahydro-1H-carbazol-3-yl)-amide; *4-Isopropoxy-3',4'-dimethoxy-biphenyl-3-carboxylic acid and Phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1')to 1% (0.25') to 1% (1.25'). Molecular peak (ESI, M+1): 516 Retention time: 4.20 min. | 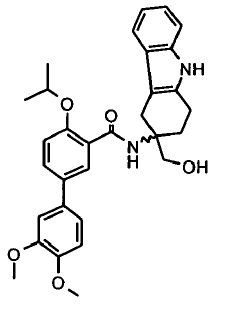 |
| **17** | 4-Isopropoxy-3',4'-dimethoxy-biphenyl-3-carboxylic acid (8-chloro-3-hydroxymethyl-2,3,4,9-tetrahydro-1H-carbazol-3-yl)-amide; *4-lsopropoxy-3',4'-dimethoxy-biphenyl-3-carboxylic acid and 2-Chloro-phenyl-hydrazine* | HPLC purification: Column X-Bridge RP C18 4.6 x 50 3.5 µM; detection wavelength 214 nm; flow rate 1 ml/min; eluents A: 0.1 % TFA in H₂O, B 0.1% TFA in ACN; gradient in each case based on B: I % to 99% (5') to 99% 1') to 1% (0.25') to 1% (1.25'). Molecular peak (ESI, M+1):550 Retention time: 4.47 min. | 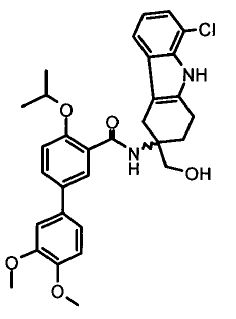 |
| **18** | 4-lsopropoxy-3',4'-dimethoxy-biphenyl-3-carboxylic acid (6-fluoro-3-hydroxymethyl-2,3,4,9-tetrahydro-1H-carbazol-3-yl)-amide; *4-Isopropoxy-3',4'-dimethoxy-biphenyl-3-carboxylic acid and 4-Fluoro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 534 Retention time: 5.88 min. | 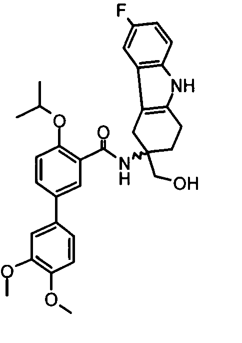 |
| **19** | 4-Isopropoxy-3',4'-dimethoxy-biphenyl-3-carboxylic acid (3-hydroxymethyl-5,8-dimethyl-2,3,4,9-tetrahydro-1H-carbazol-3-yl)-amide; *4-Isopropoxy-3', 4'-dimethoxy-biphenyl-3-carboxylic acid and 2,5-Dimethyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 544 Retention time: 4.53 min. | 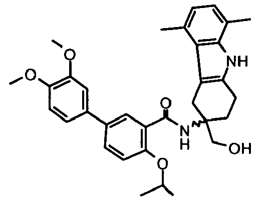 |
| **20** | 4-Isopropoxy-3',4'-dimethoxy-biphenyl-3-carboxylic acid (3-hydroxymethyl-6-trifluoromethyl-2,3,4,9-tetrahydro-1H-carbazol-3-yl)-amide; *4-Isopropoxy-3',4'-dimethoxy-biphenyl-3-carboxylic acid and 4- Trifluoromethyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 584 Retention time: 4.47 min. | 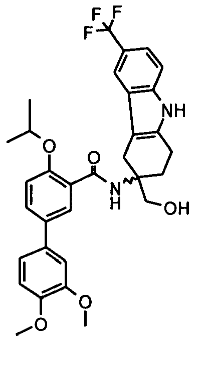 |
| **21** | 4-Isopropoxy-3',4'-dimethoxy-biphenyl-3-carboxylic acid (3-hydroxymethyl-6-trifluoromethoxy-2,3,4,9-tetrahydro-1H-carbazol-3-yl)-amide; *4-lsopropoxy-3',4'-dimethoxy-biphenyl-3-carboxylic acid and 4-Trifluoromethyloxy-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 600 Retention time: 4.53 min. | 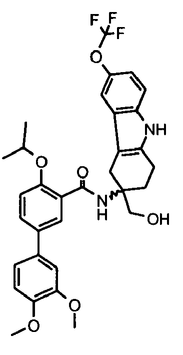 |
| **22** | 4-Isopropoxy-3',4'-dimethoxy-biphenyl-3-carboxylic acid (8-fluoro-3-hydroxymethyl-2,3,4,9-tetrahydro-1H-carbazol-3-yl)-amide; *4-Isopropoxy-3,4'-dimethoxy-biphenyl-3-carboxylic acid and 2-Fluoro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 534 Retention time: 4.30 min. | 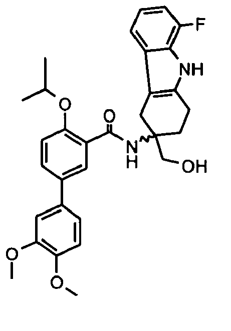 |
| **23** | N-(3-Hydroxymethyl-8-trifluoromethyl-2,3,4,9-tetrahydro-1H-carbazol-3-yl)-2-isopropoxy-5-(2-methoxy-phenylethynyl)-benzamide; *2-Isopropoxy-5-(2-methoxy-phenylethynyl)-benzoic acid and 2- Trifluoromethyl-phenyl-hyrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 578 Retention time: 6.47 min. | 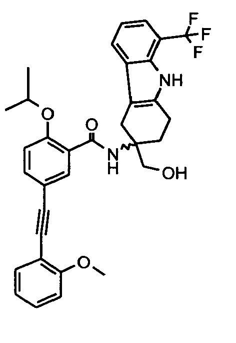 |
| **24** | N-(3-Hydroxymethyl-2,3,4,9-tetrahydro-1H-carbazol-3-yl)-2-isopropoxy-5-(2-methoxy-henylethynyl)-benzamide; *2-Isopropoxy-5-(2-methoxy-phenylethynyl)-benzoic acid and Phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 510 Retention time: 4.57 min. | 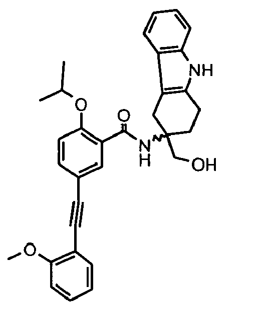 |
| **25** | 3'-Chloro-4-isopropoxy-biphenyl-3,4'-dicarboxylic acid 3-[(5,8-dichloro-3-hydroxymethyl-2,3,4,9-tetrahydro-1 H-carbazol-3-yl)-amide] 4'-methyl-amide; *3'-Chloro-4-isopropoxy-4'-methylcarbamoyl-biphenyl-3-carboxylic a-cid and 2, 5-Dichloro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 616 Retention time: 4.32 min. | 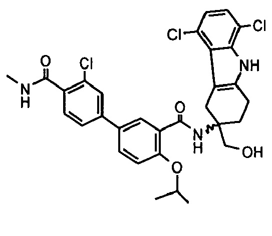 |
| **26** | 3'-Chloro-4-isopropoxy-biphenyl-3,4'-dicarboxylic acid 3-[(3-hydroxymethyl-8-trifluoromethyl-2,3,4,9-tetrahydro-1 H-carbazol-3-yl)-amide] 4'-methyl-amide; *3'-Chloro-4-isopropoxy-4'-methylcarbamoyl-biphenyl-3-carboxylic a-cid and 2-Trifluoromethyl-phenyl-hydrazine* | HPLC purification: Column X-Bridge RP C18 4.6 x 50 3.5 µM; detection wavelength 214 nm; flow rate 1 ml/min; eluents A: 0.1 % TFA in H₂O, B 0.1% TFA in ACN; gradient in each case based on B: 1% to 99% (5') to 99% (1') to 1% (0.25') to 1 % (1.25'). Molecular peak (ESI, M+1): 615 Retention time: 4.19 min. | 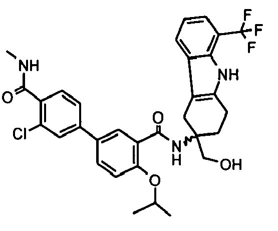 |
| **27** | 3'-Chloro-4-isopropoxy-biphenyl-3,4'-dicarboxylic acid 3-[(3-hydroxymethyl-2,3,4,9-tetrahydro-1 H-carbazol-3-yl)-amide] 4'-methylamide; *3'-Chloro-4-isopropoxy-4'-methylcarbamoyl-biphenyl-3-carboxylic a-cid and Phenyl-hydrazine* | HPLC purification: Column X-Bridge RP C18 4.6 x 50 3.5 µM; detection wavelength 214 nm; flow rate 1 ml/min; eluents A: 0.1 % TFA in H₂O, B 0.1% TFA in ACN; gradient in each case based on B: 1% to 99% (5') to 99% (1') to 1% (0.25') to 1% (1.25'). Molecular peak (ESI, M+1): 547 Retention time: 5.43 min. | 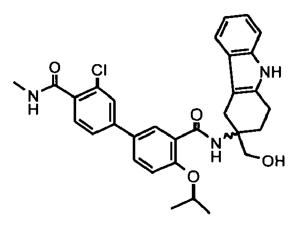 |
| **28** | 6-[(3'-Chloro-4-isopropoxy-4'-methylcarbamoyl-biphenyl-3-carbonyl)-amino]-6-hydroxymethyl-6,7,8,9-tetrahydro-5H-carbazole-1-carboxylic acid; *3'-Chloro-4-isopropoxy-4'-methylcarbamoyl-biphenyl-3-carboxylic a-cid and 2-Carboxy-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 591 Retention time: 3.41 min. | 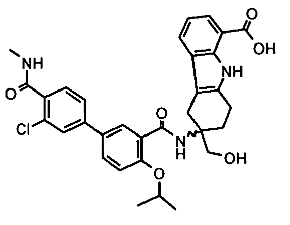 |
| **29** | 6-[(3'-Chloro-4-isopropoxy-4'-methylcarbamoyl-biphenyl-3-carbonyl)-amino]-6-hydroxymethyl-6,7,8,9-tetrahydro-5H-carbazole-3-carboxylic acid; *3'-Chloro-4-isopropoxy-4 '-methylcarbamoyl-biphenyl-3-carboxylic a-cid and 4-Carboxy-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 591 Retention time: 3.20 min. | 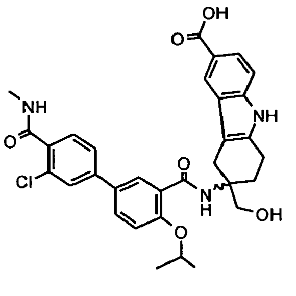 |
| **30** | 3'-Chloro-4-isopropoxy- biphenyl-3,4'-dicarboxylic acid 3-[(8-chloro-3-hydroxymethyl-2,3,4,9-tetrahydro-1H-carbazol-3-yl)-amide] 4'- methylamide; *3'-Chloro-4-isopropoxy-4'-methylcarbamoyl-biphenyl-3-carboxylic a-cid 2-Chloro-phenyl-hydrazine* | I ( HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 582 Retention time: 4.09 min. | 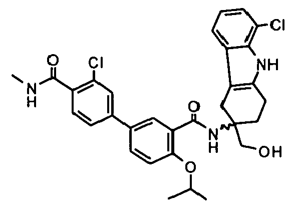 |
| **31** | 3'-Chloro-4-isopropoxy-biphenyl-3,4'-dicarboxylic acid 3-[(5-chloro-3-hydroxymethyl-6-methyl-2,3,4,9-tetrahydro-1H-carbazol-3-yl)-amide] 4'-methylamide; *3'-Chloro-4-isopropoxy-4'-methylcarbamoyl-biphenyl-3-carboxylic a-cid and 3-Chloro-4-methyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 596 Retention time: 5.91 min. | 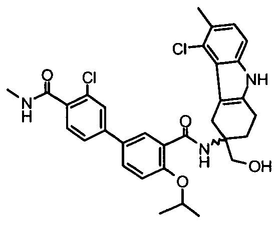 |
| **32** | 3'-Chloro-4-isopropoxy-biphenyl-3,4'-dicarboxylic acid 3-[(3-hydroxymethyl-6-methyl-2,3,4,9-tetrahydro-1H-carbazol-3-yl)-amide] 4'-methylamide; *3'-Chloro-4-isopropoxy-4'-methylcarbamoyl-biphenyl-3-carboxylic a-cid and 4-Methyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 561 Retention time: 4.00 min. | 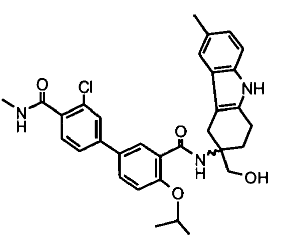 |
| **33** | 3'-Chloro-4-isopropoxy-biphenyl-3,4'-dicarboxylic acid 3-[(6-fluoro-3-hydroxymethyl-2,3,4,9-tetrahydro-1H-carbazol-3-yl)-amide] 4'-methylamide; *3'-Chloro-4-isopropoxy-4'-methylcarbamoyl-biphenyl-3-carboxylic a-cid and 4-Fluoro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 565 Retention time: 3.85 min. | 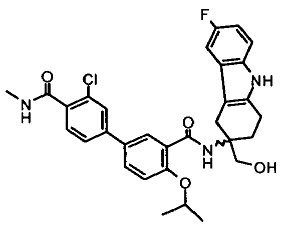 |
| **34** | 3'-Chloro-4-isopropoxy-biphenyl-3,4'-dicarboxylic acid 3-[(6-chloro-3-hydroxymethyl-2,3,4,9-tetrahydro-1 H-carbazol-3-yl)-amide] 4'-methylamide; *3'-Chloro-4-isopropoxy-4'-methylcarbamoyl-biphenyl-3-carboxylic a-cid and 4-Chloro-phenyl-hydrazine* | HPLC purification: Column X-Bridge RP C18 4.6 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 582 Retention time: 4.05 min. | 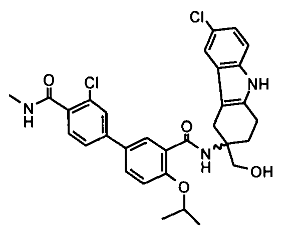 |
| **35** | 3'-Chloro-4-isopropoxy-biphenyl-3,4'-dicarboxylic acid 3-[(7-chloro-6-fluoro-3-hydroxymethyl-2,3,4,9-tetrahydro-1 H-carbazol-3-yl)-amide] 4'-methylamide; *3'-Chloro-4-isopropoxy-4'-methylcarbamoyl-biphenyl-3-carboxylic a-cid and 3-Chloro-4-fluoro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1 % TFA in ACN; gradient in each case based on B: 1% to 99% (5') to 99% (1') to 1% (0.25') to 1 % (1.25'). Molecular peak (ESI, M+1): 599 Retention time: 4.05 min. | 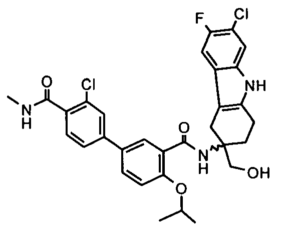 |
| **36** | 5-Benzofuran-2-yl-N-(5,8-dichloro-3-hydroxymethyl-2,3,4,9-tetrahydro-1H-carbazol-3-yl)-2-isopropoxy-benzamide; *5-Benzofuran-2-yl-2-isopropoxy-benzoic acid and 2, 5-Dichloro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 564 Retention time: 5.25 min. | 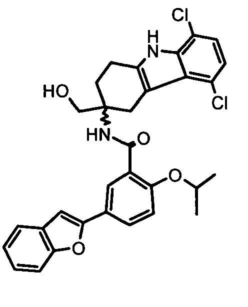 |
| **37** | 5-Benzofuran-2-yl-N-(3-hydroxymethyl-8-trifluoromethyl-2,3,4,9-tetrahydro-1H-carbazol-3-yl)-2-isopropoxy-benzamide; *5-Benzofuran-2-yl-2-isopropoxy-benzoic acid and 2-Trifluoromethyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 564 Retention time: 5.11 min. | 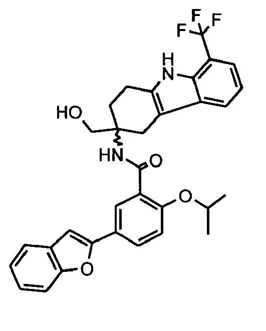 |
| **38** | 5-Benzofuran-2-yl-N-(3-hydroxymethyl-2,3,4,9-tetrahydro-1H-carbazol-3-yl)-2-isopropoxy-benzamide; *5-Benzofuran-2-yl-2-isopropoxy-benzoic acid and Phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1):496 Retention time: 4.83 min. | 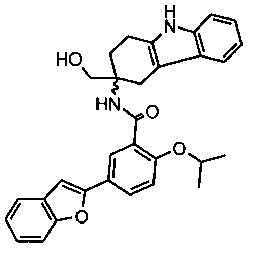 |
| **39** | 6-(5-Benzofuran-2-yl-2-isopropoxy-benzoylamino)-6-hydroxymethyl-6,7,8,9-tetrahydro-5H-carbazole-3-carboxylic acid; *5-Benzofuran-2-yl-2-isopropoxy-benzoic acid and 4-Carboxy-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1% (1.25'). Molecular peak (ESI, M+1): 540 Retention time: 4.02 min. | 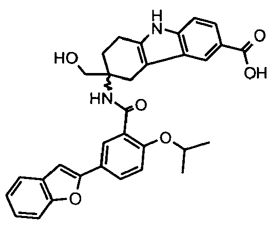 |
| **40** | 5-Benzofuran-2-yl-N-(5-chloro-3-hydroxymethyl-6-methyl-2,3,4,9-tetrahydro-1H-carbazol-3-yl)-2-isopropoxy-benzamide; *5-Benzofuran-2-yl-2-isopropoxy-benzoic acid and 3-Chloro-4-methyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 544 Retention time: 5.18 min. | 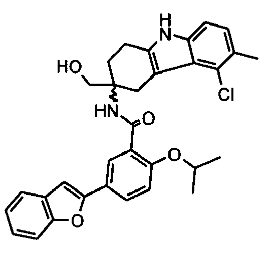 |
| **41** | 5-Benzofuran-2-yl-N-(3-hydroxymethyl-6-methyl-2,3,4,9-tetrahydro-1H-carbazol-3-yl)-2-isopropoxy-benzamide; *5-Benzofuran-2-yl-2-isopropoxy-benzoic acid and 4-Methyl-phenyl-hydrazine* | HPLC purification: Column X-Bridge RP C18 4.6 x 50 3.5 µM; detection wavelength 214 nm; flow rate 1 ml/min; eluents A: 0.1 % TFA in H₂O, B 0.1% TFA in ACN; gradient in each case based on B: 1% to 99% (5') to 99% (1') to 1% (0.25') to 1% (1.25'). Molecular peak (ESI, M+1): 510 Retention time: 4.94 min. | 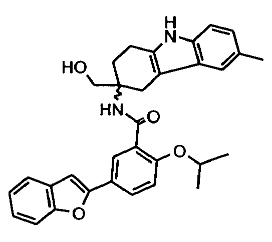 |
| **42** | 5-Benzofuran-2-yl-N-(6-fluoro-3-hydroxymethyl-2,3,4,9-tetrahydro-1H-carbazol-3-yl)-2-isopropoxy-benzamide; *5-Benzofuran-2-yl-2-isopropoxy-benzoic acid and 4-Fluoro-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 514 Retention time: 4.73 min. | 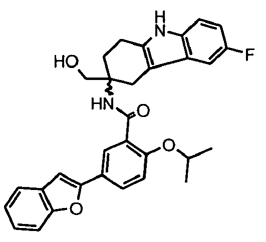 |
| **43** | 5-Benzofuran-2-yl-N-(6-chloro-3-hydroxymethyl-8-methyl-2,3,4,9-tetrahydro-1H-carbazol-3-yl)-2-isopropoxy-benzamide; *5-Benzofuran-2-yl-2-isopropoxy-benzoic acid and 4-Chloro-2-methyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 544 Retention time: 5.18 min. | 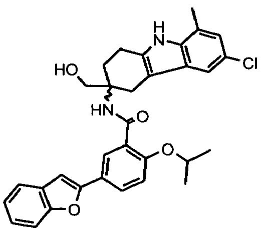 |
| **44** | 5-Benzofuran-2-yl-N-(3-hydroxymethyl-5, 8-dimethyl-2,3,4,9-tetrahydro-1H-carbazol-3-yl)-2-isopropoxy-benzamide; *5-Benzofuran-2-yl-2-isopropoxy-benzoic acid and 2, 5-Dimethyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 524 Retention time: 5.07 min. | 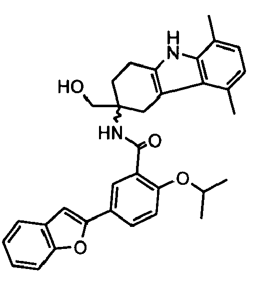 |
| **45** | 5-Benzofuran-2-yl-N-(3-hydroxymethyl-5,7-dimethyl-2,3,4,9-tetrahydro-1H-carbazol-3-yl)-2-isopropoxy-benzamide; *5-Benzofuran-2-yl-2-isopropoxy-benzoic acid and 3, 5-Dimethyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1% TFA in ACN; gradient in each case based on B: 1% to 99% (5') to 99% (1') to 1% (0.25') to 1 % (1.25'). Molecular peak (ESI, M+1): 524 Retention time: 5.12 min. | 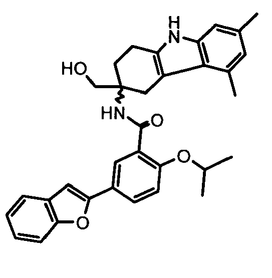 |
| **46** | 5-Benzofuran-2-yl-N-(3-hydroxymethyl-8-methyl-2,3,4,9-tetrahydro-1H-carbazol-3-yl)-2-isopropoxy-benzamide; *5-Benzofuran-2-yl-2-isopropoxy-benzoic acid 2-Methyl-phenyl-hydrazine* | HPLC purification: Column X-Bridge RP C18 4.6 x 50 3.5 µM; detection wavelength 214 nm; flow rate 1 ml/min; eluents A: 0.1% TFA in H₂O, B 0.1 % TFA in ACN; gradient in each case based on B: 1% to 99% (5') to 99% (1') to 1% (0.25') to 1 % (1.25'). Molecular peak (ESI, M+1): 510 Retention time: 4.99 min. | 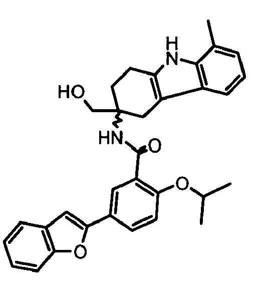 |

## Claims

1. 2,3,4,9-tetrahydro-1H-carbazoles of the formula I 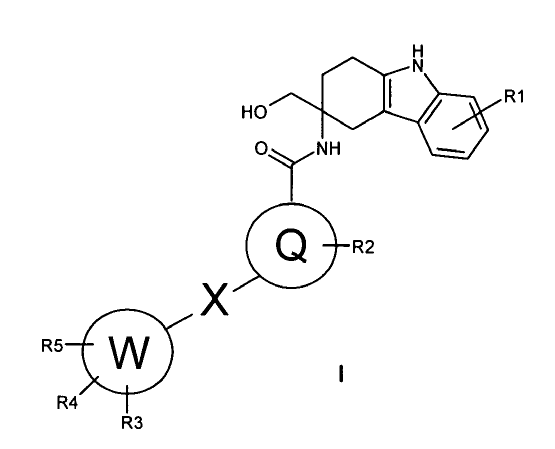 in which
R1 is hydrogen, halogen, cyano, -SO₂Me, carboxy, -SO₂NH₂, -SO₃H, nitro, amino, C₁-C₆-alkyl, C₂-C₆-alkenyl, C₂-C₆-alkynyl or C₁-C₆-alkyloxy, where the hydrocarbon chains therein may optionally be substituted one or more times by fluorine, cyano or hydroxy;
R2 is hydrogen, hydroxy, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkylamino-C₁-C₆-alkylene, di(C₁-C₆-alkyl)amino-C₁-C₆-alkylene;
where the hydrocarbon chains therein may optionally be substituted one or more times by fluorine, cyano, hydroxy, amino
or by the groups: 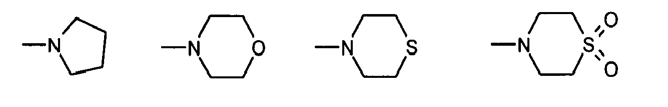 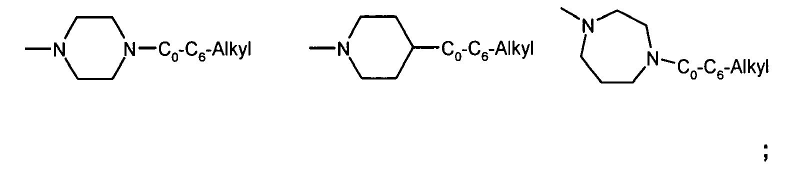
R3, R4, R5 are independently of one another hydrogen, hydroxy, halogen, nitro, amino, cyano, phenyl, C₁-C₆-alkyl, C₂-C₆-alkenyl or C₂-C₆-alkynyl, C₃-C₇-cycloalkyl, C₃-C₇-cycloalkyl-C₁-C₆-alkylene, C₃-C₇-heterocycloalkyl, where the hydrocarbon chains therein may optionally be substituted one or more times by fluorine, cyano or by the radicals: 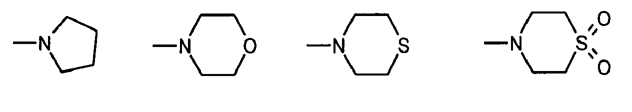 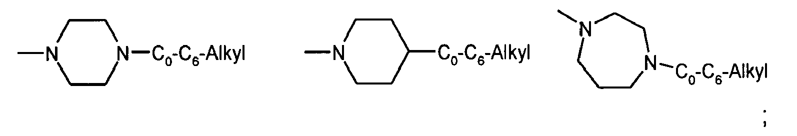 or
R3, R4, R5 independently of one another hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, phenyloxy-C₁-C₆-alkylene, C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino, C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneamino-carbonyl, C₁-C₆-alkylcarbonyl, C₃-C₇-cycloalkylcarbonyl, carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl, -N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl, -C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine, -S(O₂)-N(H)-C₂C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine, -O-C₂-C₆-alkylene-(C₁-C₆-alkyl)amine, -O-C₂-C₆-alkylene-[di(C₁-C₆-alkylene)]amine,
or the radicals: 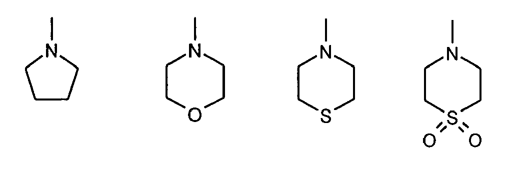 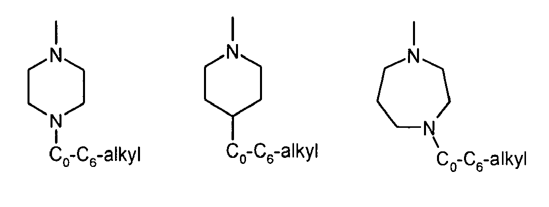 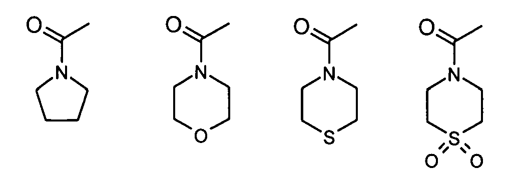 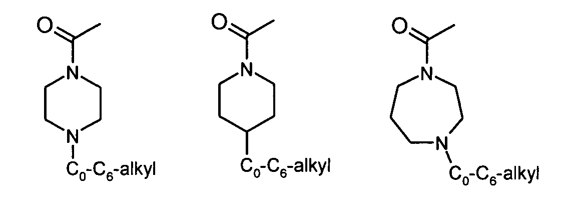 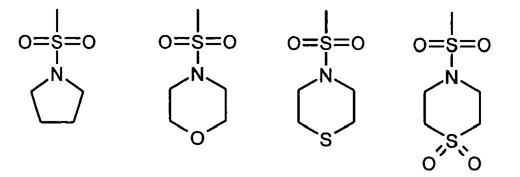 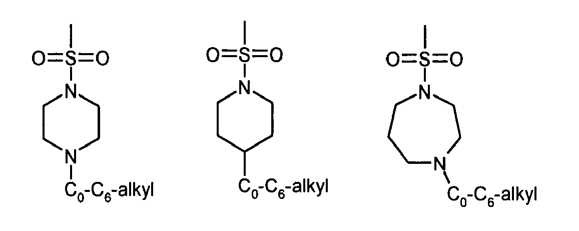 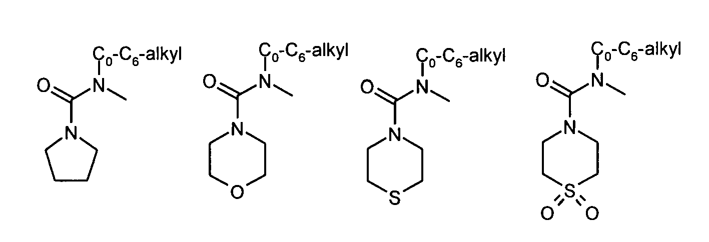 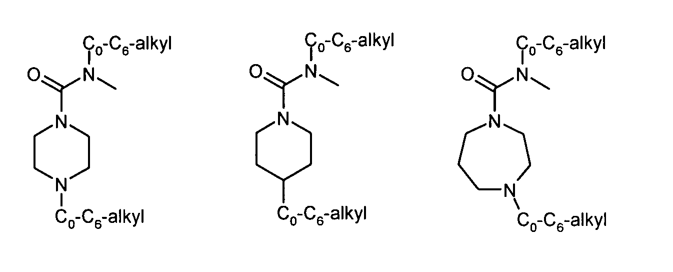 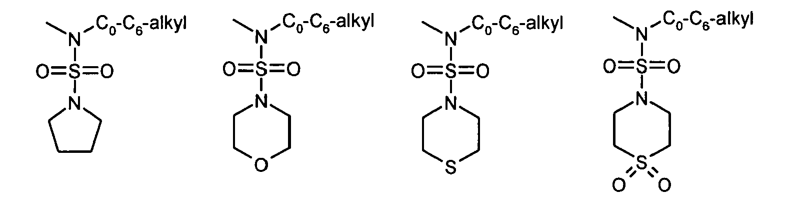 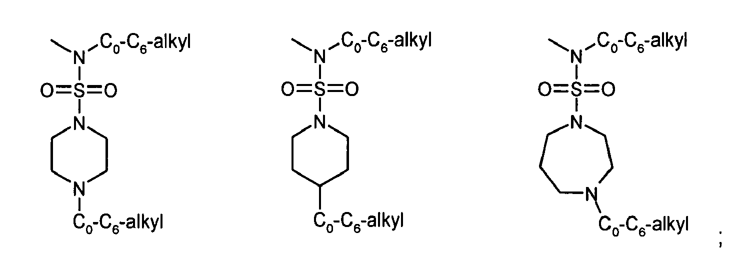
R3 and R4 may together form heterocycloalkyl, cycloalkyl;
Q is an aryl or heteroaryl group
or the group 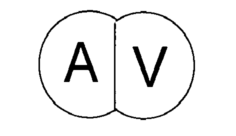 in which
A is a monocyclic aryl or a monocyclic heteroaryl group;
V is a cycloalkylen, cycloalkenylen, heterocycloalkylen or heterocycloalkenylen group;
X is a bond, C₁-C₄-alkylene, C₂-C₄-alkenylene, C₂-C₄-alkynylene;
W is an aryl or heteroaryl group;
where
R1 may substitute one or more positions of the aryl ring in the tetrahydrocarbazole moiety;
R2 may substitute one or more positions of the aryl or heteroaryl ring in the radical Q or in the radical V.

2. Compounds according to claim 1, namely 2,3,4,9-tetrahydro-1H-carbazoles of the formula II 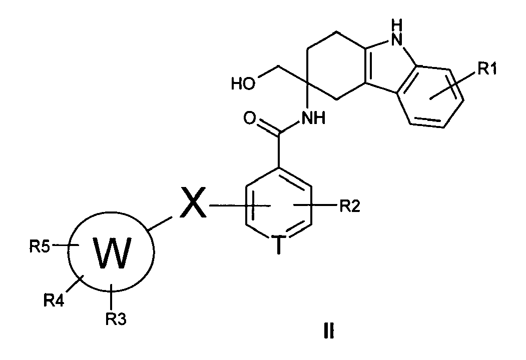 wherein
T is a nitrogen atom or a CH group; and
R1, R2, R3, R4, R5, X, and W have the same meaning as defined in claim 1.

3. Compounds according to claim 1, namely 2,3,4,9-tetrahydro-1H-carbazoles of the formula III 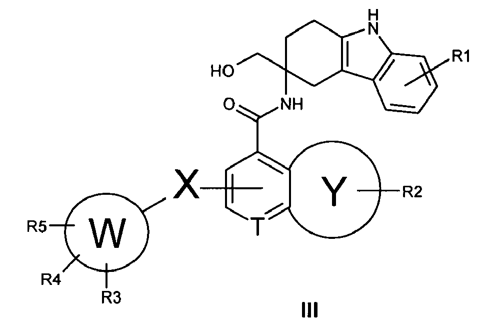 wherein
T is a nitrogen atom or a CH group;
Y is a monocyclic aryl or a monocyclic heteroaryl group or a cycloalkylen, cycloalkenylen, heterocycloalkylen or heterocycloalkenylen group;
and
R1, R2, R3, R4, R5, X and W have the same meaning as defined in claim 1,
where
R2 may substitute one or more positions of the aryl or heteroaryl ring in the radical Y.

4. Compounds according to claims 1 or 2, namely 2,3,4,9-tetrahydro-1H-carbazoles of the formula IV 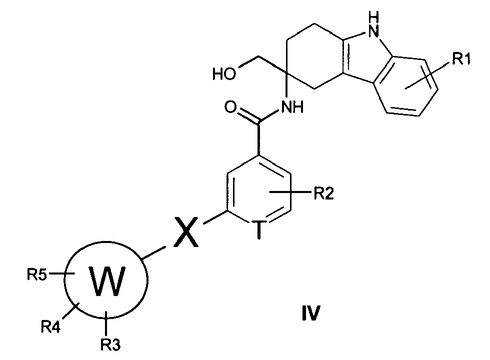 wherein
T is a nitrogen atom or a CH group; and
R1, R2, R3, R4, R5, X, and W have the same meaning as defined in claim 1.

5. Compounds according to claims 1 or 3, namely 2,3,4,9-tetrahydro-1H-carbazoles of the formula V 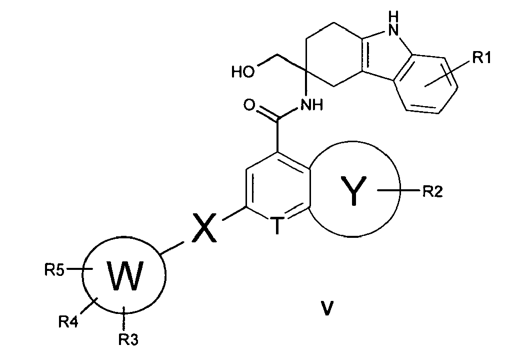 wherein
T is a nitrogen atom or a CH group;
Y is a monocyclic aryl or a monocyclic heteroaryl group or a cycloalkylen, cycloalkenylen, heterocycloalkylen or heterocycloalkenylen group;
and
R1, R2, R3, R4, R5, X and W have the same meaning as defined in claim 1;
where
R2 may substitute one or more positions of the aryl or heteroaryl ring in the radical Y.

6. Compounds according to any of the preceding claims, namely
**1** 6-Methoxy-2-(3,4,5-trimethoxy-phenyl)-quinoline-4-carboxylic acid ((S)-3-hydroxymethyl-2,3,4,9-tetrahydro-1 H-carbazol-3-yl)-amide;
**2** 6-Methoxy-2-phenyl-quinoline-4-carboxylic acid (5,8-dichloro-3-hydroxymethyl-2,3,4,9-tetrahydro-1H-carbazol-3-yl)-amide;
**3** 6-Methoxy-2-phenyl-quinoline-4-carboxylic acid (3-hydroxymethyl-8-trifluoromethyl-2,3,4,9-tetrahydro-1H-carbazol-3-yl)-amide;
**4** 6-Methoxy-2-phenyl-quinoline-4-carboxylic acid (3-hydroxymethyl-2,3,4,9-tetrahydro-1H-carbazol-3-yl)-amide;
**5** 6-Hydroxymethyl-6-[(6-methoxy-2-phenyl-quinoline-4-carbonyl)-amino]-6,7,8,9-tetrahydro-5H-carbazole-3-carboxylic acid;
**6** 6-Methoxy-2-phenyl-quinoline-4-carboxylic acid (8-chloro-3-hydroxymethyl-2,3,4,9-tetrahydro-1H-carbazol-3-yl)-amide;
**7** 6-Methoxy-2-phenyl-quinoline-4-carboxylic acid (5-chloro-3-hydroxymethyl-6-methyl-2,3,4,9-tetrahydro-1H-carbazol-3-yl)-amide;
**8** 6-Methoxy-2-phenyl-quinoline-4-carboxylic acid (6-chloro-3-hydroxymethyl-8-methyl-2,3,4,9-tetrahydro-1H-carbazol-3-yl)-amide;
**9** 6-Methoxy-2-phenyl-quinoline-4-carboxylic acid (3-hydroxymethyl-5,8-dimethyl-2,3,4,9-tetrahydro-1H-carbazol-3-yl)-amide;
**10** 6-Methoxy-2-phenyl-quinoline-4-carboxylic acid (3-hydroxymethyl-6-trifluoromethyl-2,3,4,9-tetrahydro-1H-carbazol-3-yl)-amide;
**11** 6-Methoxy-2-phenyl-quinoline-4-carboxylic acid (3-hydroxymethyl-6-trifluoro-methoxy-2,3,4,9-tetrahydro-1H-carbazol-3-yl)-amide;
**12** 6-Methoxy-2-phenyl-quinoline-4-carboxylic acid (8-fluoro-3-hydroxymethyl-2,3,4,9-tetrahydro-1H-carbazol-3-yl)-amide;
**13** 6-Methoxy-2-phenyl-quinoline-4-carboxylic acid (7-chloro-6-fluoro-3-hydroxymethyl-2,3,4,9-tetrahydro-1H-carbazol-3-yl)-amide;
**14** 6-Methoxy-2-phenyl-quinoline-4-carboxylic acid (3-hydroxymethyl-6-sulfamoyl-2,3,4,9-tetrahydro-1 H-carbazol-3-yl)-amide;
**15** 6-Methoxy-2-phenyl-quinoline-4-carboxylic acid (5,6-dichloro-3-hydroxymethyl-2,3,4,9-tetrahydro-1H-carbazol-3-yl)-amide;
**16** 4-lsopropoxy-3',4'-dimethoxy-biphenyl-3-carboxylic acid (3-hydroxymethyl-2,3,4,9-tetrahydro-1 H-carbazol-3-yl)-amide;
**17** 4-lsopropoxy-3',4'-dimethoxy-biphenyl-3-carboxylic acid (8-chloro-3-hydroxymethyl-2,3,4,9-tetrahydro-1 H-carbazol-3-yl)-amide;
**18** 4-Isopropoxy-3',4'-dimethoxy-biphenyl-3-carboxylic acid (6-fluoro-3-hydroxymethyl-2,3,4,9-tetrahydro-1 H-carbazol-3-yl)-amide;
**19** 4-Isopropoxy-3',4'-dimethoxy-biphenyl-3-carboxylic acid (3-hydroxymethyl-5,8-dimethyl-2,3,4,9-tetrahydro-1H-carbazol-3-yl)-amide;
**20** 4-Isopropoxy-3',4'-dimethoxy-biphenyl-3-carboxylic acid (3-hydroxymethyl-6-trifluoromethyl-2,3,4,9-tetrahydro-1H-carbazol-3-yl)-amide;
**21** 4-Isopropoxy-3',4'-dimethoxy-biphenyl-3-carboxylic acid (3-hydroxymethyl-6-trifluoromethoxy-2,3,4,9-tetrahydro-1H-carbazol-3-yl)-amide;
**22** 4-Isopropoxy-3',4'-dimethoxy-biphenyl-3-carboxylic acid (8-fluoro-3-hydroxymethyl-2,3,4,9-tetrahydro-1H-carbazol-3-yl)-amide;
**23** N-(3-Hydroxymethyl-8-trifluoromethyl-2,3,4,9-tetrahydro-1H-carbazol-3-yl)-2-isopropoxy-5-(2-methoxy-phenylethynyl)-benzamide;
**24** N-(3-Hydroxymethyl-2,3,4,9-tetrahydro-1H-carbazol-3-yl)-2-isopropoxy-5-(2-methoxy-henylethynyl)-benzamide;
**25** 3'-Chloro-4-isopropoxy-biphenyl-3,4'-dicarboxylic acid 3-[(5,8-dichloro-3-hydroxymethyl-2,3,4,9-tetrahydro-1H-carbazol-3-yl)-amide] 4'-methylamide;
**26** 3'-Chloro-4-isopropoxy-biphenyl-3,4'-dicarboxylic acid 3-[(3-hydroxymethyl-8-trifluoromethyl-2,3,4,9-tetrahydro-1H-carbazol-3-yl)-amide]4'-methylamide;
**27** 3'-Chloro-4-isopropoxy-biphenyl-3,4'-dicarboxylic acid 3-[(3-hydroxymethyl-2,3,4,9-tetrahydro-1H-carbazol-3-yl)-amide] 4'-methylamide;
**28** 6-[(3'-Chloro-4-isopropoxy-4'-methylcarbamoyl-biphenyl-3-carbonyl)-amino]-6-hydroxymethyl-6,7,8,9-tetrahydro-5H-carbazole-1-carboxylic acid;
**29** 6-[(3'-Chloro-4-isopropoxy-4'-methylcarbamoyl-biphenyl-3-carbonyl)-amino]-6-hydroxymethyl-6,7,8,9-tetrahydro-5H-carbazole-3-carboxylic acid;
**30** 3'-Chloro-4-isopropoxy-biphenyl-3,4'-dicarboxylic acid 3-[(8-chloro-3-hydroxymethyl-2,3,4,9-tetrahydro-1 H-carbazol-3-yl)-amide] 4'-methylamide;
**31** 3'-Chloro-4-isopropoxy-biphenyl-3,4'-dicarboxylic acid 3-[(5-chloro-3-hydroxymethyl-6-methyl-2,3,4,9-tetrahydro-1H-carbazol-3-yl)-amide] 4'-methylamide;
**32** 3'-Chloro-4-isopropoxy-biphenyl-3,4'-dicarboxylic acid 3-[(3-hydroxymethyl-6-methyl-2,3,4,9-tetrahydro-1 H-carbazol-3-yl)-amide] 4'-methylamide;
**33** 3'-Chloro-4-isopropoxy-biphenyl-3,4'-dicarboxylic acid 3-[(6-fluoro-3-hydroxymethyl-2,3,4,9-tetrahydro-1 H-carbazol-3-yl)-amide] 4'-methylamide;
**34** 3'-Chloro-4-isopropoxy-biphenyl-3,4'-dicarboxylic acid 3-[(6-chloro-3-hydroxymethyl-2,3,4,9-tetrahydro-1H-carbazol-3-yl)-amide] 4'-methylamide;
**35** 3'-Chloro-4-isopropoxy-biphenyl-3,4'-dicarboxylic acid 3-[(7-chloro-6-fluoro-3-hydroxymethyl-2,3,4,9-tetrahydro-1H-carbazol-3-yl)-amide] 4'-methylamide;
**36** 5-Benzofuran-2-yl-N-(5,8-dichloro-3-hydroxymethyl-2,3,4,9-tetrahydro-1H-carbazol-3-yl)-2-isopropoxy-benzamide;
**37** 5-Benzofuran-2-yl-N-(3-hydroxymethyl-8-trifluoromethyl-2,3,4,9-tetrahydro-1H-carbazol-3-yl)-2-isopropoxy-benzamide;
**38** 5-Benzofuran-2-yl-N-(3-hydroxymethyl-2,3,4,9-tetrahydro-1H-carbazol-3-yl)-2-isopropoxy-benzamide;
**39** 6-(5-Benzofuran-2-yl-2-isopropoxy-benzoylamino)-6-hydroxymethyl-6,7,8,9-tetrahydro-5H-carbazole-3-carboxylic acid;
**40** 5-Benzofuran-2-yl-N-(5-chloro-3-hydroxymethyl-6-methyl-2,3,4,9-tetrahydro-1 H-carbazol-3-yl)-2-isopropoxy-benzamide:
**41** 5-Benzofuran-2-yl-N-(3-hydroxymethyl-6-methyl-2,3,4,9-tetrahydro-1H-carbazol-3-yl)-2-isopropoxy-benzamide;
**42** 5-Benzofuran-2-yl-N-(6-fluoro-3-hydroxymethyl-2,3,4,9-tetrahydro-1H-carbazol-3-yl)-2-isopropoxy-benzamide;
**43** 5-Benzofuran-2-yl-N-(6-chloro-3-hydroxymethyl-8-methyl-2,3,4,9-tetrahydro-1 H-carbazol-3-yl)-2-isopropoxy-benzamide;
**44** 5-Benzofuran-2-yl-N-(3-hydroxymethyl-5,8-dimethyl-2,3,4,9-tetrahydro-1H-carbazol-3-yl)-2-isopropoxy-benzamide;
**45** 5-Benzofuran-2-yl-N-(3-hydroxymethyl-5,7-dimethyl-2,3,4,9-tetrahydro-1H-carbazol-3-yl)-2-isopropoxy-benzamide;
**46** 5-Benzofuran-2-yl-N-(3-hydroxymethyl-8-methyl-2,3,4,9-tetrahydro-1H-carbazol-3-yl)-2-isopropoxy-benzamide.

7. Process for preparing compounds of the formula I of claim 1, wherein a tetrahydrocarbazol derivative VI 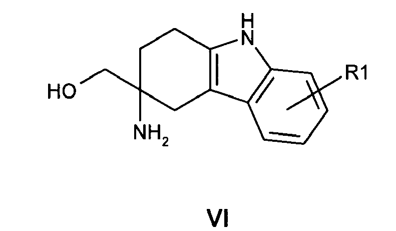 in which the radical R1 has the same meaning as defined in claim 1,
is coupled with a carboxylic acid of the formula VII 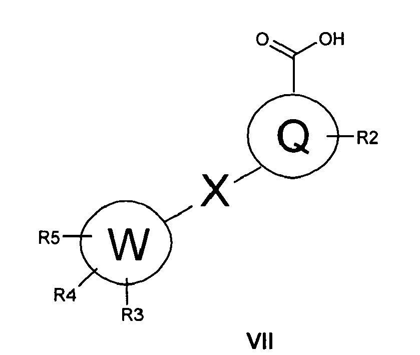 in which R2, R3, R4, R5, Q, X and W have the same meaning as defined in claim 1,
in an amide forming reaction comprising
a) conversion of said carboxylic acid into an intermediate active ester or carbonyl chloride with a suitable peptide-coupling reagent, or with thionyl chloride, oxalyl chloride, phosgene or derivatives thereof, where appropriate in the presence of a base,
b) reacting the active intermediate resulting from step a) with said tetrahydrocarbazol.

8. Process according to claim 7 for preparing compounds of the formula II of claim 2, wherein a tetrahydrocarbazol derivative of the formula VI is coupled with a carboxylic acid of the formula VIII 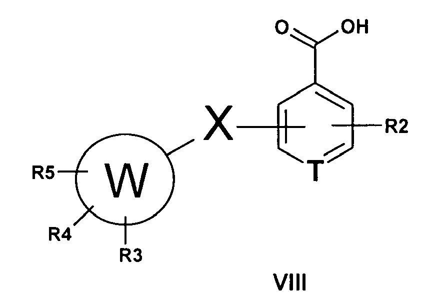 in which R2, R3, R4, R5, T, X and W have the same meaning as defined in claim 2.

9. Process according to claim 7 for preparing compounds of the formula III of claim 3, wherein a tetrahydrocarbazol derivative of the formula VI is coupled with a carboxylic acid of the formula IX 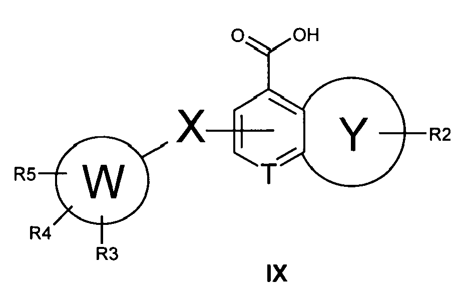 in which R2, R3, R4, R5, T, X, Y and W have the same meaning as defined in claim 3.

10. Process according to claim 7 for preparing compounds of the formula IV of claim 4, wherein a tetrahydrocarbazol derivative of the formula VI is coupled with a carboxylic acid of the formula X 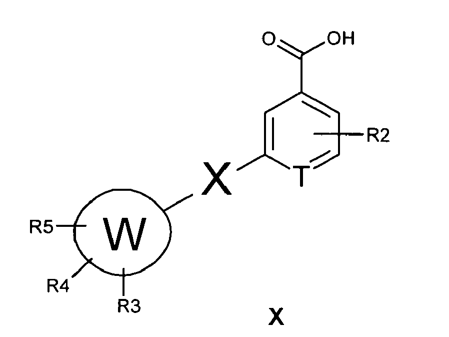 in which R2, R3, R4, R5, T, X and W have the same meaning as defined in claim 4.

11. Process according to claim 7 for preparing compounds of the formula V of claim 5, wherein a tetrahydrocarbazol derivative of the formula VI is coupled with a carboxylic acid of the formula XI 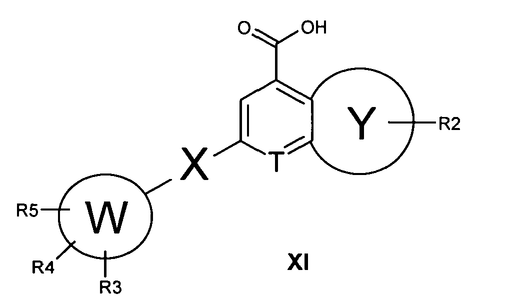 in which R2, R3, R4, R5, T, X, Y and W have the same meaning as defined in claim 5.

12. Process for preparing compounds of the formula I of claim 1, comprising
a) conversion of an acetal derivative of the formula XIII 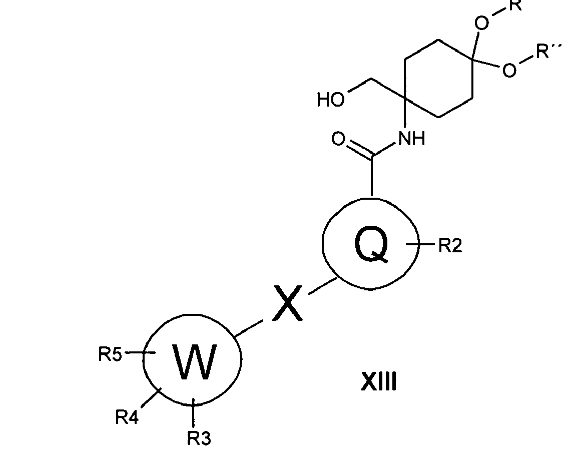 in which R2, R3, R4, R5, Q, X, W have the same meaning as defined in claim 1, and
R' and R" are C₁-C₆-alkyl, or together form a cyclic acetal, or are linked/bonded to a solid phase resin,
into the corresponding ketone in the presence of an inorganic/organic protic or Lewis acid,
b) optionally isolating the intermediate ketone resulting from step a), and
c) reacting the ketone resulting from step a) or b),
where appropriate, in the presence of an inorganic/organic protic or Lewis acid,
with a phenyl hydrazine of the formula XII 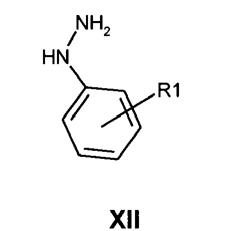 in which R1 has the same meaning as defined in claim 1.

13. Process according to claim 12 for preparing compounds of the formula II of claim 2, wherein the acetal derivative in step a) is of the formula XIV 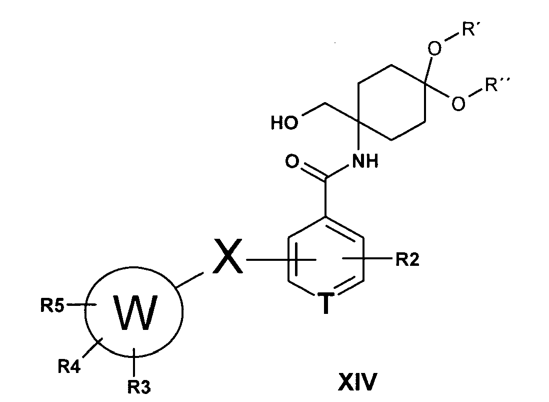 in which R2, R3, R4, R5, T, X and W have the same meaning as defined in claim 2, and
R' and R" are C₁-C₆-alkyl, or together form a cyclic acetal, or are linked/bonded to a solid phase resin.

14. Process according to claim 12 for preparing compounds of the formula III of claim 3 wherein the acetal derivative in step a) is of the formula XV 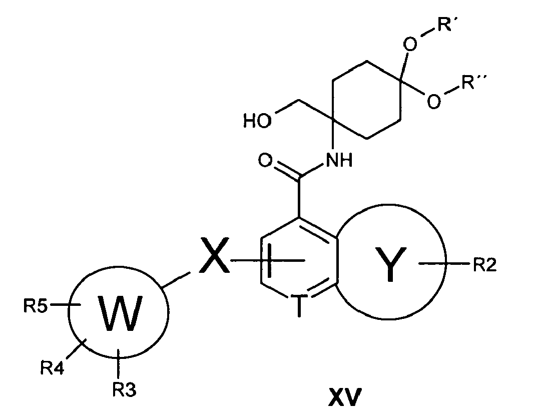 in which R2, R3, R4, R5, T, X, Y and W have the same meaning as defined in claim 3, and
R' and R" are C₁-C₆-alkyl, or together form a cyclic acetal, or are linked/bonded to a solid phase resin.

15. Process according to claim 12 for preparing compounds of the formula IV of claim 4, wherein the acetal derivative in step a) is of the formula XVI 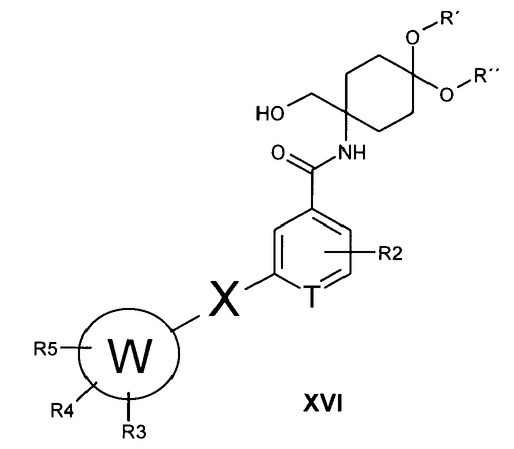 in which R2, R3, R4, R5, T, X and W have the same meaning as defined in claim 4, and
R' and R" are C₁-C₆-alkyl, or together form a cyclic acetal, or are linked/bonded to a solid phase resin.

16. Process according to claim 12 for preparing compounds of the formula V of claim 5, wherein the acetal derivative in step a) is of the formula XVII 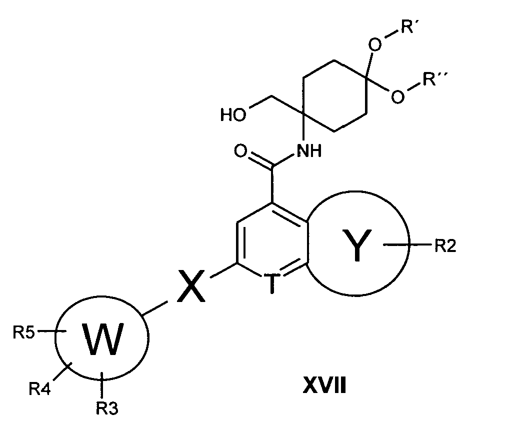 in which R2, R3, R4, R5, T, X and W have the same meaning as defined in claim 5, and
R' and R" are C₁-C₆-alkyl, or together form a cyclic acetal, or are linked/bonded to a solid phase resin.

17. Pharmaceutical compositions comprising at least one of the compounds according to any of claims 1 to 6 with pharmaceutically suitable excipients and/or carriers.

18. Use of the compounds of the general formula I according to any of claims 1 to 6 for the fertility control in men or in women.

19. Process for producing medicaments comprising at least one of the compounds of the general formula I according to any of claims 1 to 6 for the prevention and/or treatment of osteoporosis.
